# EUROPEAN PATENT APPLICATION

(11) **EP 4 726 057 A1**
(43) Date of publication of application: **15.04.2026**
(21) Application number: 24205630.7
(22) Date of filing: 09.10.2024
(51) Int. Cl.: C12Q 1/6883

(54) **IN VITRO METHOD FOR IDENTIFYING THE RISK OF DEVELOPING GESTATIONAL DIABETES MELLITUS**

(71) Applicant: Universidad de los Andes, 7620086 Santiago (CL)
(72) Inventor: CHAPARRO PADILLA, Alejandra, 7620086 Las Condes, Santiago (CL); NARDOCCI VALENZUELA, Gino, 7620086 Las Condes, Santiago (CL); ILLANES LÓPEZ, Sebastián, 7620086 Las Condes, Santiago (CL)
(74) Representative: Hoffmann Eitle

(57) **Abstract**

The present invention provides a *vitro* method for screening or identifying pregnant females at risk of developing gestational diabetes mellitus (GDM), the method comprising the steps of:
(a) measuring the nucleic acid expression levels of one or more RNAs in a gingival crevicular fluid (GCF) sample obtained from a pregnant female to be screened, and
(b) comparing the RNA expression levels obtained in (a) with the expression levels of the same one or more RNA measured in a control GCF sample and determining that the pregnant female screened in (a) is at risk of having gestational diabetes mellitus if the RNA expression levels measured in (a) are deviated or different compared to the RNA expression levels measured in the control GCF sample.

## Description

### TECHNICAL FIELD

The present invention refers to the medical field. Particularly, it refers to methods based on the assessment of screening methods to detect whether a pregnant female is at risk of developing gestational diabetes mellitus.

### BACKGROUND ART

Gestational diabetes mellitus (GDM) is defined as various degrees of glucose intolerance with first recognition during pregnancy. In 2010, the American Association of Diabetes (ADA) and the International Association of Diabetes and Pregnancy Study Groups (IADPSG) proposed new screening and diagnostic criteria for diabetes in pregnancy. Using these criteria, the global prevalence of hyperglycemia in pregnancy has been estimated to be 17%, with regional estimates varying between 10% and 25% in North America and Southeast Asia, respectively.

Although GDM usually resolves within 6 weeks of delivery, approximately 50% of women diagnosed with this metabolic disorder are expected to develop type 2 diabetes mellitus (T2DM) over a 10-30-year period. Women with GDM also experience an increased risk of developing other pregnancy complications, such as preeclampsia, and their offspring are at higher risk of developing short-term adverse outcomes such as macrosomia, neonatal hypoglycemia, and neonatal cardiac dysfunction, and long-term complications, such as obesity, impaired glucose tolerance (IGT), and diabetes in adolescence or early adulthood.

The criteria for the diagnosis of GDM recommend universal screening for GDM at 24-28 weeks of gestation using the oral glucose tolerance test (OGTT). Although this is diagnostically robust, it has the disadvantage of not allowing early intervention in patients who develop diabetes as pregnancy progresses. If an effective 1st-trimester screening test is available, the damage accumulated during the clinically occult phase (i.e. up to 24-28 weeks) could be averted by early intervention. The prediction of patients at risk of developing GDM during the first stages of pregnancy would provide an opportunity for timely management that could improve outcomes for both the mother and the baby.

Currently, no early pregnancy tests are clinically available to detect women at a risk of developing GDM. Although studies have shown that some blood-borne biomarkers can provide a good negative predictive measure for subsequent GDM, most tests have poor positive predictive values and limited clinical efficacy highlighting the need for new approaches to accomplish this task.

In recent years, RNA, and particular non-coding RNAs (ncRNAs), have gained importance as regulators of gene expression in various biological processes in humans and other organisms. These RNA molecules are currently a hot topic in biological research, owing to their essential regulatory mechanisms in mammalian homeostasis and pathogenesis. The regulation of ncRNAs has been associated with crucial roles in many fundamental biological processes, such as development, metabolism, immunity, cancer, and epigenetic control, among many others. Because of the essential role of ncRNAs in gene regulation, they have been highlighted as potential biomarkers for the diagnosis and prognosis of various pathologies, making them good candidates as biomarkers.

The present invention provides reliable biomarkers for the identification of pregnant females that are at risk of developing gestational diabetes mellitus.

### BRIEF DESCRIPTION OF DRAWINGS

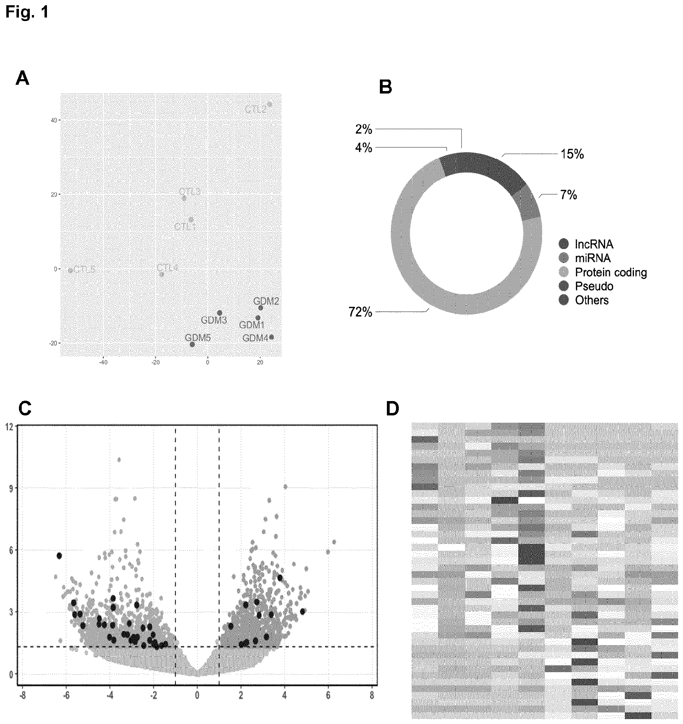

**Figure 1****:** RNA-seq and Differential Gene Expression Analysis in Control and GDM Patients. A) Principal Component Analysis (PCA) of the Control and GDM Samples. B) Donut Chart Illustrating Major Gene Classes Identified by Sequencing. C) Volcano Plot Displaying Differentially Expressed Genes with IncRNAs Highlighted in Blue. Vertical dotted lines indicate log2 fold change (FC) >1 or ≤-1, while the horizontal dotted line represents a p-value <0.05. Green and orange spots denote the upregulated and downregulated genes, respectively. D) Heatmap of Identified IncRNA candidates (12 upregulated in green bars and 32 downregulated in orange bars). The normalized counts are shown in a heatmap.

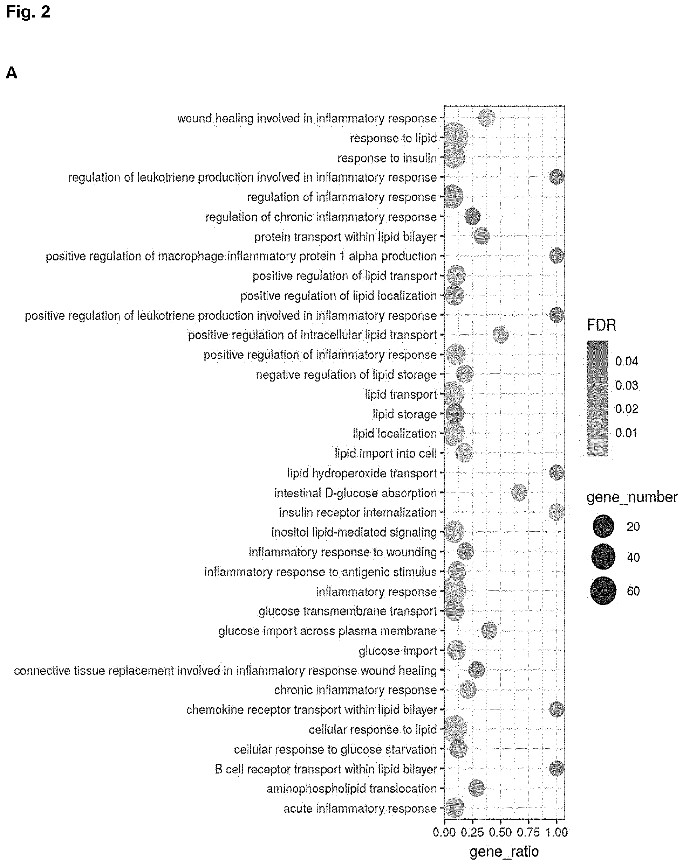

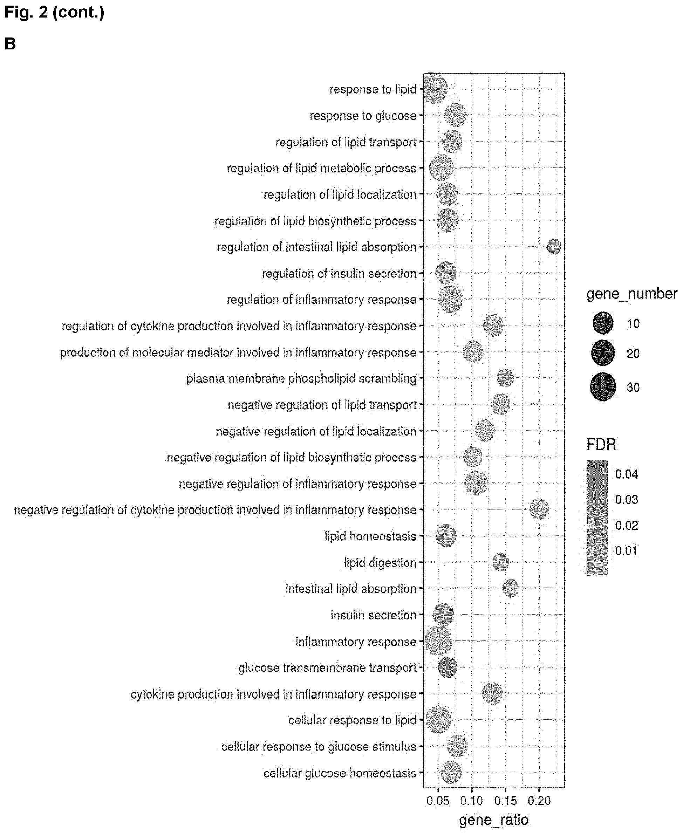

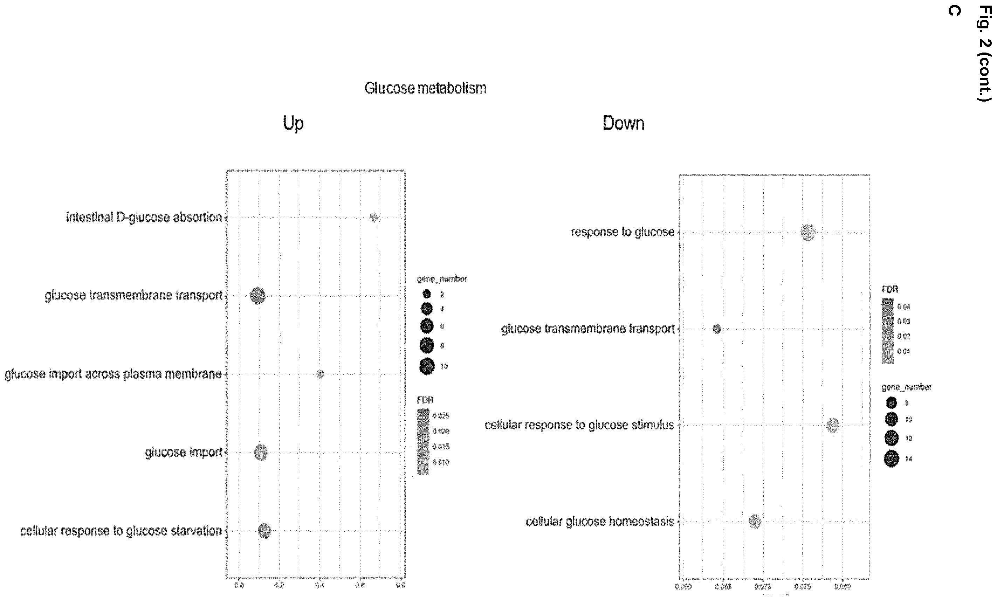

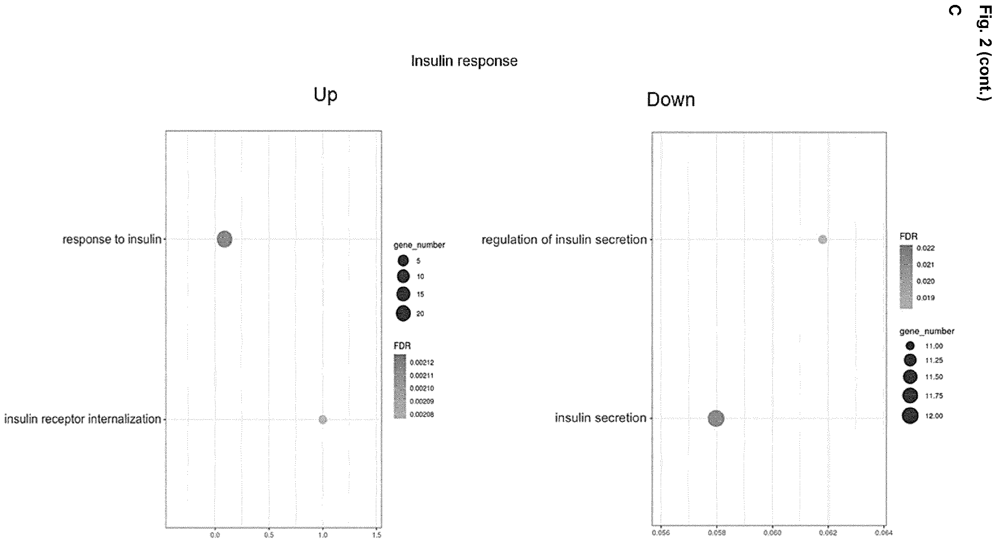

**Figure 2****.** Functional annotations and over-representation analysis of the most enriched and down enriched GO molecular functions, of the exclusive and differentially expressed RNAs respectively (A and B). GO analyses of most representative RNAs genes involved in glucose metabolism and insulin response. C) Volcano plot showing differentially expressed genes, with IncRNAs Highlighted in Blue. Vertical dotted lines indicate log2 fold change (FC) ≥1 or ≤-1, whereas the horizontal dotted line represents a p-value <0.05. Green and orange spots denote the upregulated and downregulated genes, respectively.

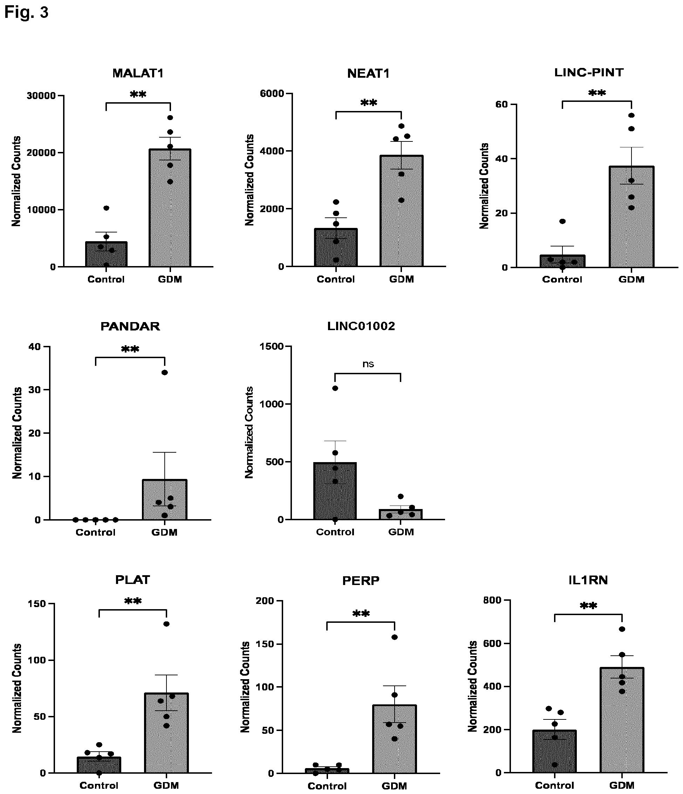

**Figure 3****.** Expression Profiles of Identified IncRNA candidates and control-coding genes from transcriptomic analysis. In this figure, the expression values of five IncRNA candidates and three coding genes designated as controls are depicted. The data were presented as normalized counts, offering insights into their expression patterns. Significance is indicated by **P ≤ 0.01, compared to the control. Results are expressed as mean ± SEM (n = 5). ns non-significant.

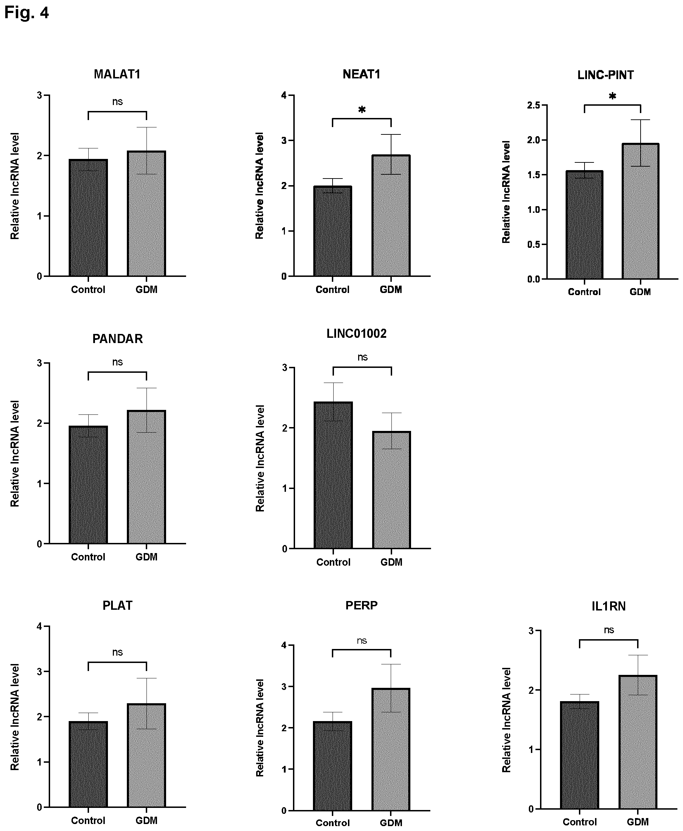

**Figure 4****.** Relative Expression of Long Non-Coding RNA (IncRNA) candidates in Gestational Diabetes Mellitus (GDM) Patients and Control Subjects. We show the relative expression of each RNA analyzed in 250 control subjects and 25 GDM patients. All the genes were normalized to U6. Significance was indicated by *P ≤ 0.05, compared to the control. Results are expressed as mean ± SEM. ns non-significant.

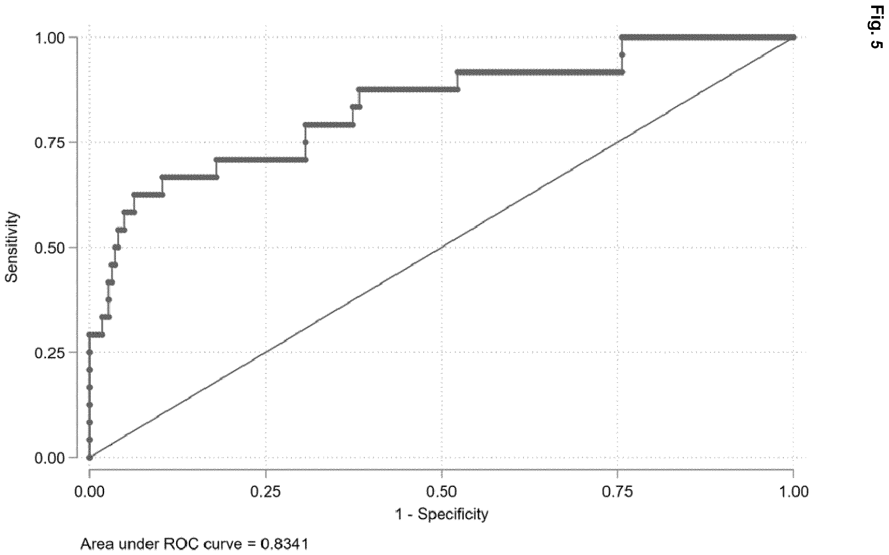

**Figure 5****.** Area under the receiver operating characteristic curve (AUC-ROC) of the logistic regression model for the prediction of GDM diagnosis at 11-14 weeks of gestation (0.83%).

### GENERAL CONSIDERATIONS AND DEFINITIONS

Each embodiment disclosed herein is contemplated as being applicable to each of the other disclosed embodiments. Thus, all combinations of the various elements described herein are within the scope of the invention. It should also be understood that, unless clearly indicated otherwise, regarding the methods of the invention that include more than one step or act, the order of the steps or acts of the method is not necessarily limited to the order in which the steps or acts of the method are mentioned.

Unless otherwise stated, the terms used in the present application have the meanings indicated herein. Terms that have not been defined herein should be given the meanings that would be given to them by a person skilled in the art in the context of this disclosure.

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

If the term "about" as used in connection with a numerical value throughout the specification and the claims denotes an interval of accuracy, familiar and acceptable to a person skilled in the art. For instance, the term "about" means the indicated value ± 1% of its value, or the term "about" means the indicated value ± 2% of its value, or the term "about" means the indicated value ± 5% of its value, the term "about" means the indicated value ± 10% of its value, or the term "about" means the indicated value ± 20% of its value, or the term "about" means the indicated value ± 30% of its value; preferably the term "about" means exactly the indicated value (± 0%).

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

The term "subject" refers to an individual or entity that is the focus of a study. In the context of the present invention, the subject is a pregnant female, preferably a pregnant woman.

### DESCRIPTION OF THE EMBODIMENTS

As explained in the background art, there is a need for the identification of reliable biomarkers for the identification of pregnant females at risk of developing gestational diabetes mellitus (GDM). Some studies have illustrated the distinct expression of ncRNAs at both placental and blood levels in individuals with gestational diabetes mellitus (GDM) when compared to control group. However, those methods are highly invasive since they require obtaining blood from the pregnant female or from placental fluid. Methods that are less invasive but maintain high predictive capacity are needed. No studies have measured these molecules in the GCF during early pregnancy in individuals at risk of developing GDM. The authors of the present invention discovered that pregnant females who will later develop GDM exhibit differential expression of RNAs in periodontal fluid. This potential variation in expression serves as a valuable biomarker for predicting diseases early in pregnancy.

In particular, the inventors of the present invention have found that the expression levels of one or more RNAs present in the GCF of a pregnant female can be used as a biomarker for assessing the risk of said pregnant female of developing GDM in later stages of pregnancy. Indeed, the inventors found a list of RNAs, as depicted in Tables 1, 2 and 6, that were either upregulated or downregulated in comparison with the GCF sample of control females that never developed GDM during or after pregnancy. Among the list of ncRNAs found with biomarker potential, the authors chose five long non coding RNAs (IncRNAs) (MALAT1, NEAT1, LINC-PINT, PANDAR, and LINC01002) for further analysis. These IncRNAs showed statistically significant differences in expression levels between the control and gestational diabetes mellitus (GDM) groups in both bioinformatics (Figure 2) and RT-qPCR validation (Figure 3) experiments. In contrast, control genes exhibited a comparable trend between their expression profiles and the validation experiments.

Lastly, the inventors performed a multivariate regression model to identify an association between the expression of RNA TPA, PERP, NEAT1, LINC-PINT, IL-1RN in GCF during the first trimester of pregnancy, combined with the initial body mass index and first trimester fasting glycemia, with the development of GDM (see Table 4A). The model was found to be a good predictor of GDM, with an ROC-AUC of 84% (95% Cl: 0.737-0.931) (Figure 2), specificity of 78.1%, sensitivity of 70.8%, PPV of 25%, and NPV of 96.3%. The positive likelihood ratio was 3.24, and the negative likelihood ratio was 0.373 (Table 4B). It is noted that TPA, PERP and IL-1RN are mRNAs, while NEAT1, PANDAR, LINC01002 and LIN-PINT are IncRNAs.

In view of the above, the present invention provides in **a first aspect** a screening method that can identify pregnant females that are at risk of developing gestational diabetes mellitus (GDM), the method comprising analysing the RNA expression profile from a gingival crevicular fluid (GCF) sample obtained from the pregnant female to be screened, and comparing said expression profile with the RNA expression profile obtained from a control GCF sample, such as a GCF sample obtained from a control female that did not develop GDM during and/or after pregnancy. A change or a deviation in the RNA expression profile of the pregnant female to be screened, above or below the RNA expression profile of a control GCF sample, is indicative that the pregnant female being screened has an increased risk of suffering GDM.

The term "gestational diabetes mellitus" or "GDM" is defined as glucose intolerance with onset or first recognition during pregnancy. The expression "Gingival crevicular fluid" or "GCF" is an inflammatory exudate that can be collected at the gingival margin or within the gingival crevice.

As used herein, the phrase "RNA expression profile" means the expression levels of one or more RNA measured from a given GCF sample. Specifically, the phrase "RNA expression profile" means a collection of measurements, such as but not limited to a quantity or concentration, for expression of individual RNAs taken from the GCF. Examples of test samples or sources of components for the RNA extracts for the gene expression profile include, but are not limited to, cells from gingival crevicular fluid or exudate from gingival crevicular fluid. It is noted that, within the term "RNA", all types of RNAs are included, such as mRNAs, miRNAs, non-coding RNAs (ncRNAs) and long non-coding RNAs (IncRNAs).

Therefore, provided herein is an *in vitro* method comprising the steps of:
(a) measuring the nucleic acid expression profile of one or more RNAs in a gingival crevicular fluid (GCF) sample obtained from a pregnant female to be screened, and
(b) comparing the RNA expression profile obtained in (a) with the expression profile of the same one or more RNA measured in a control GCF sample and determining that the pregnant female screened in (a) is at risk of having gestational diabetes mellitus if the RNA expression profile measured in (a) is different or deviated compared to the RNA expression profile measured in the control GCF sample.

The term "screening" is understood as the examination or testing of a pregnant female or a group of pregnant females pertaining to the general population, at risk of suffering from GDM, with the objective of discriminating healthy individuals from those who are suffering from an undiagnosed GDM or who are at high risk of suffering from said indication. Thus, the expression "pregnant female that is at risk of having gestational diabetes mellitus" refers to a pregnant female who has a higher likelihood or probability of presenting a GDM than a control female. The methods herein proposed "for the identification of a pregnant female that is at risk of having gestational diabetes mellitus" may be used to filter pregnant females that are susceptible or suspected of developing GDM. If the pregnant female is identified as to have an increased risk of having GDM, it may be beneficial to verify the diagnosis of the disease with additional tests or to provide a treatment to her. The increased risk may be relative or absolute and may be expressed qualitatively or quantitatively. For example, an increased risk may be expressed as simply determining the pregnant female `s RNA expression profile and placing the pregnant female in an "increased risk" category, based upon previous population studies. Alternatively, a numerical expression of the pregnant female `s increased risk may be determined based upon the RNA expression profile. As used herein, examples of expressions of an increased risk include but are not limited to, odds, probability, odds ratio, p-values, attributable risk, relative frequency, positive predictive value, negative predictive value, and relative risk.

Thus, the method of the present invention comprises measuring the RNA expression profile of one or more RNA(s) present in a GCF sample obtained from a pregnant female, and determining if there is a differential RNA expression profile as compared to the expression levels of the same one or more RNA measured in a control GCF sample. Techniques to assay RNA expression levels from a given sample are well known to the skilled technician, and the invention is not limited by the means by which they are assessed. In one embodiment, RNA expression levels are assessed using RNA sequencing or quantitative arrays, and the like. To determine the level of RNA expression, complete RNA sequencing information can be, but is not necessarily, used to determine quantitative levels of RNA expression, and thereby a RNA expression profile. In addition, to determine levels of RNA expression, it is not necessary that an entire RNA transcript be present or fully sequenced. In other words, determining levels of, for example, a fragment of an RNA transcript analysed may be sufficient to conclude or assess that the RNA being analysed is up- or down-regulated. Similarly, if, for example, arrays or blots are used to determine RNA expression levels, the presence/absence/strength of a detectable signal will be sufficient to assess levels of RNA expression without the need to sequencing an RNA transcript.

The RNA expression levels can be expressed as absolute or relative values and may or may not be expressed in relation to another component, a standard, such an internal standard, or another molecule of compound known to be in the sample. If the levels are assessed as relative to a standard or internal standard, the standard may be added to the test sample prior to, during or after sample processing. To assess RNA expression levels a GCF sample is taken from the pregnant female to be screened. The GCF sample may or may not be processed prior assaying RNA expression levels.

The expression "differential or deviated RNA expression profile as compared to the RNA expression profile obtained in a control GCF sample" refers to a difference or deviation in the expression levels of one or more RNA(s) measured in the GCF of the pregnant female to be screened, in comparison to the same one or more RNA(s) expression levels measured in control GCF sample. This deviation may be an upregulation of the RNA expression levels or a downregulation of the RNA expression levels. In the context of this invention, the difference or deviation is preferably significant according to statistical methods, or is a deviation of at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79% or 80% as compared to the RNA expression levels of a control GCF sample.

In an embodiment, the RNA expression levels measured in (a) are decreased in comparison to the expression levels of the same RNA measured in a control GCF sample. The term "decrease(d)" refers to a reduction that is significant according to statistical methods, or to a reduction of at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79% or 80% as compared to the RNA expression levels measured in control GCF sample.

In an embodiment, the RNA expression levels measured in (a) are increased in comparison to the expression levels of the same RNA measured in a control GCF sample. The term "increase(d)" refers to a growth that is significant according to statistical methods, or to a growth of at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79% or 80% as compared to the RNA expression levels measured in control GCF sample.

In an embodiment, the one or more RNA(s) measured in step (a) are down-regulated in comparison to the same RNA measured in a control GCF sample. The term "down-regulated" of any of the RNA measured in step (a) described in the present invention, preferably refers to a decrease in their expression level by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more with respect to the expression levels of the same RNA measured in a control GCF sample.

In an embodiment, the one or more RNA(s) measured in step (a) are up-regulated comparison to the same RNA measured in a control GCF sample. The term "up-regulated" of any of the RNA measured in step (a) described in the present invention, preferably refers to an increase in their expression level by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more with respect to the expression levels of the same RNA measured in a control GCF sample.

Preferably, the one or more RNA(s) measured in step (a) have a statistically significant increased expression in comparison to the same one or more RNA(s) measured in a control GCF sample. By "statistically significant increased expression" is referred herein as the determination by an analyst that the increase in the expression levels is not explainable by chance alone. In an embodiment, the RNAs whose expression levels are increased or upregulated, preferably statistically increased, in a GCF sample in comparison to the levels of the same RNAs in a control GCF sample are one or more selected from the list consisting of NEAT1, LINC00673, MIR205HG, MALAT1, PP7080, RUNX1-IT1, LINC-PINT, LINC00342, LRRC75A-AS1, PSMA3-AS1, RDH10-AS1, PANDAR, or any combination thereof (see Table 6, left column). In an embodiment, the RNAs whose expression levels are increased or upregulated, preferably statistically increased, in a GCF sample in comparison to the levels of the same RNAs in a control GCF sample are one or more selected from the list consisting of NEAT1, MALAT1, LINC-PINT, PLAT, PERP, IL1RN, PANDAR, or any combination thereof (see Fig 3). In an embodiment, the RNAs whose expression levels are increased or upregulated, preferably statistically increased, in a GCF sample in comparison to the levels of the same RNAs in a control GCF sample are one or more selected from the list consisting of LINC-PINT, NEAT1, TPA, PERP, and IL-1RN. In an embodiment, the RNAs whose expression levels are increased or upregulated, preferably statistically increased, in a GCF sample in comparison to the levels of the same RNAs in a control GCF sample are one or more selected from the list consisting of TPA, PERP, and LINC-PINT. In an embodiment, the RNAs whose expression levels are increased or upregulated, preferably statistically increased, in a GCF sample in comparison to the levels of the same RNAs in a control GCF sample are one or more selected from the list consisting of NEAT1 and LINC-PINT.

Preferably, the one or more RNA(s) measured in step (a) have a statistically significant reduced expression in comparison to the same one or more RNA(s) measured in a control GCF sample. By "statistically significant reduced expression" is referred herein as the determination by an analyst that the reduction in the expression levels is not explainable by chance alone. In an embodiment, the RNAs whose expression levels are reduced or down-regulated, preferably statistically reduced, in a GCF sample in comparison to the levels of the same RNAs in a control GCF sample are one or more selected from the list consisting of LINC01166, LOC285696, SUCLG2-AS1, C15orf32, C9orf135-AS1, LINC00960, FLJ33534, LINC01604, C2orf91, FLJ16779, MORC2-AS1, LINC01002, C7orf65, CTBP1-AS, TMEM105, EPHA1-AS1, SOCS2-AS1, VPS9D1-AS1, C9orf139, LOC729737, EMX2OS, LINC01314, RRN3P2, LOC101928093, WDFY3-AS2, LOC221946, LOC101929567, LOC100506022, LOC100288152, MAP3K14, LOC100129697, LINC00324, or any combination thereof (see Table 6, right column). Preferably, the RNA that is reduced, preferably statistically reduced, is LINC01002.

Statistical hypothesis testing is the method by which the skilled person makes this determination. This test provides a p-value, which is the probability of observing results as extreme as those in the data, assuming the results are truly due to chance alone. A p-value of 0.1 or lower (preferably 0.05, 0.01, 0.001 or lower) is considered herein to be statistically significant. For example, the increase in the expression of the of a given RNA in a GCF sample isolated from a pregnant female is statistically significant when a statistical test is performed to compare it to the expression levels of said RNA in a reference GCF sample, and wherein the resulting p-value of said statistical test is of 0.1 or lower, preferably 0.05, 0.01, 0.001 or lower. Preferably, the p value is lower than 0.5.

Thus, the RNA expression levels measured in step (a) are compared in step (b) to the expression levels of the same RNA measured in a control GCF sample. The control GCF sample may be obtained from a control female, or it may be a pre-established threshold value. The term "control female" preferably refers to a reference female, or a group thereof, that did not develop GDM when they were pregnant nor after giving birth. The term "pre-established threshold value" refers to a specific, predetermined numerical limit or boundary set in advance, which is used as a criterion to trigger a certain action, decision, or response when it is reached or exceeded. Preferably, pre-established threshold value refers to already established RNA expression levels or profile from females that did not suffer from GDM during and after pregnancy.

This threshold is typically defined based on historical data, theoretical considerations, regulatory requirements, or expert judgment, and serves as a benchmark for monitoring and evaluating various parameters or conditions. In the context of this invention, a pre-established threshold value might be a specific expression level of one or more RNAs that, when breached, indicates that the pregnant female that is being screened has a risk of suffering from gestational diabetes mellitus. Thus, when referring to the expression levels of the RNAs described in the present invention, a pre-established threshold value refers to a reference RNAs expression level indicative that a pregnant female being screened is likely to suffer from gestational diabetes mellitus with a given sensitivity and specificity if the expression levels of the patient are above or below said pre-established threshold value.

A variety of statistical and mathematical methods for establishing the threshold or cutoff level of expression are known in the prior art. A threshold or cutoff expression level for a particular biomarker may be selected, for example, based on data from Receiver Operating Characteristic (ROC) plots, as described in the Examples of the present invention. One of skill in the art will appreciate that these threshold or cutoff expression levels can be varied, for example, by moving along the ROC plot for a particular biomarker or combinations thereof, to obtain different values for sensitivity or specificity thereby affecting overall assay performance. For example, if the objective is to have a robust diagnostic method from a clinical point of view, we should try to have a high sensitivity. However, if the goal is to have a cost-effective method we should try to get a high specificity. The best cutoff refers to the value obtained from the ROC plot for a particular biomarker that produces the best sensitivity and specificity. Sensitivity and specificity values are calculated over the range of thresholds (cutoffs). Thus, the threshold or cutoff values can be selected such that the sensitivity and/or specificity are at least about 70 %, and can be, for example, at least 75 %, at least 80 %, at least 85 %, at least 90 %, at least 95 %, at least 96 %, at least 97 %, at least 98 %, at least 99 % or at least 100% in at least 60 % of the patient population assayed, or in at least 65 %, 70 %, 75 % or 80 % of the patient population assayed.

In an embodiment, the RNA whose expression levels are measured in step (a) include, but are not limited to, the ncRNAs LINC01166, SUCLG2-AS1, C9orf135-AS1, FLJ33534, C2orf91, MORC2-AS1, C7orf65, TMEM105, SOCS2-AS1, C9orf139, EMX2OS, RRN3P2, WDFY3-AS2, LOC101929567, LOC100288152, LOC100129697, LOC285696, C15orf32, LINC00960, LINC01604, FLJ16779, LINC01002, NEAT1, LINC00673, MIR205HG, MALAT1, PP7080, RUNX1-IT1, LINC-PINT, LINC00342, LRRC75A-AS1, PSMA3-AS1, RDH10-AS1, PANDAR, MIR16-2, MIR199B, MIR339, MIR185, MIR16-1, MIR454, MIR25, MIR183, MIR766, MIR1249, MIR652, MIR148A, MIR505, MIR425, MIR424, MIR223, MIR345, MIR93, MIR6821, MIR361, MIR590, MIR7-1, MIR106B, MIR30E, MIR187, MIR193A, MIR15B, MIR186, MIR34A, MIR130A, MIR146A, MIR8055, MIR197, MIR628, MIR142, MIR324, MIR26B, MIR23A, MIR421, MIR132, MIR191, MIR96, MIR3613, MIR30D, MIR328, MIR30B, MIR532, MIR320A, MIRLET7D, MIRLET7I, MIR21, MIR4449, MIR221, MIR29A, MIRLET7G, MIR708, MIR200C, MIR222, MIR342, MIR205, MIR210, MIR193B, MIR574, MIR99A, MIR27A, MIR5047, MIR15A, MIR140, and/or MIR145. If two RNAs are used in generating the RNAs expression profile, any combination of two or more RNAs listed above can be used. Preferably, step (a) of the method of the present invention comprises measuring the RNA expression levels of at least one of the RNAs selected from Table 1 or 6 or 8.

**Table 1:ncRNA candidates**

| **Official gene symbols** | **ncRNA type** | **Official gene symbols** | **ncRNA type** |
|---|---|---|---|
| LINC01166 | IncRNA | MIR425 | miRNA |
| SUCLG2-AS1 | IncRNA | MIR424 | miRNA |
| C9orf135-AS1 | IncRNA | MIR223 | miRNA |
| FLJ33534 | IncRNA | MIR345 | miRNA |
| C2orf91 | IncRNA | MIR93 | miRNA |
| MORC2-AS1 | IncRNA | MIR6821 | miRNA |
| C7orf65 | IncRNA | MIR361 | miRNA |
| TMEM105 | IncRNA | MIR590 | miRNA |
| SOCS2-AS1 | IncRNA | MIR7-1 | miRNA |
| C9orf139 | IncRNA | MIR106B | miRNA |
| EMX2OS | IncRNA | MIR30E | miRNA |
| RRN3P2 | IncRNA | MIR187 | miRNA |
| WDFY3-AS2 | lncRNA | MIR193A | miRNA |
| LOC101929567 | lncRNA | MIR15B | miRNA |
| LOC100288152 | lncRNA | MIR186 | miRNA |
| LOC100129697 | lncRNA | MIR34A | miRNA |
| LOC285696 | lncRNA | MIR130A | miRNA |
| C15orf32 | lncRNA | MIR146A | miRNA |
| LlNC00960 | lncRNA | MIR8055 | miRNA |
| LINC01604 | lncRNA | MIR197 | miRNA |
| FLJ16779 | lncRNA | MIR628 | miRNA |
| LINC01002 | lncRNA | MIR142 | miRNA |
| CTBP1-AS | lncRNA | MIR324 | miRNA |
| EPHA1-AS1 | lncRNA | MIR26B | miRNA |
| VPS9D1-AS1 | lncRNA | MIR23A | miRNA |
| LOC729737 | lncRNA | MIR421 | miRNA |
| LlNC01314 | lncRNA | MIR132 | miRNA |
| LOC101928093 | lncRNA | MIR191 | miRNA |
| LOC221946 | lncRNA | MIR96 | miRNA |
| LOC100506022 | lncRNA | MIR3613 | miRNA |
| MAP3K14 | lncRNA | MIR30D | miRNA |
| LINC00324 | lncRNA | MIR328 | miRNA |
| N EAT1 | lncRNA | MIR30B | miRNA |
| LINC00673 | lncRNA | MIR532 | miRNA |
| MIR205HG | lncRNA | MIR320A | miRNA |
| MALAT1 | lncRNA | MIRLET7D | miRNA |
| PP7080 | lncRNA | MIRLET71 | miRNA |
| RUNX1-IT1 | lncRNA | MIR21 | miRNA |
| LINC-PINT | IncRNA | MIR4449 | miRNA |
| LINC00342 | IncRNA | MIR221 | miRNA |
| LRRC75A-AS1 | IncRNA | MIR29A | miRNA |
| PSMA3-AS1 | IncRNA | MIRLET7G | miRNA |
| RDH10-AS1 | IncRNA | MIR708 | miRNA |
| PANDAR | IncRNA | MIR200C | miRNA |
| MIR16-2 | miRNA | MIR222 | miRNA |
| MIR199B | miRNA | MIR342 | miRNA |
| MIR339 | miRNA | MIR205 | miRNA |
| MIR185 | miRNA | MIR210 | miRNA |
| MIR16-1 | miRNA | MIR193B | miRNA |
| MIR454 | miRNA | MIR574 | miRNA |
| MIR25 | miRNA | MIR99A | miRNA |
| MIR183 | miRNA | MIR27A | miRNA |
| MIR766 | miRNA | MIR5047 | miRNA |
| MIR1249 | miRNA | MIR15A | miRNA |
| MIR652 | miRNA | MIR140 | miRNA |
| MIR148A | miRNA | MIR145 | miRNA |
| MIR505 | miRNA | | |

**Table 6.- IncRNAs candidates**

| **Up-Regulated Official gene symbols** | **Down-Regulated Official gene symbols** | |
|---|---|---|
| NEAT1 | LINC01166 | LOC285696 |
| LINC00673 | SUCLG2-AS1 | C15orf32 |
| MIR205HG | C9orf135-AS1 | LINC00960 |
| MALAT1 | FLJ33534 | LINC01604 |
| PP7080 | C2orf91 | FLJ16779 |
| RUNX1-IT1 | MORC2-AS1 | LINC01002 |
| LINC-PINT | C7orf65 | CTBP1-AS |
| LINC00342 | TMEM105 | EPHA1-AS1 |
| LRRC75A-AS1 | SOCS2-AS1 | VPS9D1-AS1 |
| PSMA3-AS1 | C9orf139 | LOC729737 |
| RDH10-AS1 | EMX2OS | LINC01314 |
| PANDAR | RRN3P2 | LOC 101928093 |
| | WDFY3-AS2 | LOC221946 |
| | LOC 101929567 | LOC100506022 |
| | LOC 100288152 | MAP3K14 |
| | LOC100129697 | LINC00324 |

It is noted that here and throughout the entire document, the RNAs mentioned herein are referred to by their official gene symbol. Their sequence and features can be obtained from https://www.ncbi.nlm.nih.gov/gene/.

Preferably, the expression levels are measured by PCR, preferably by qRT-PCR. Preferably, the levels of IncRNA MALAT1 are measured by PCR, preferably qRT-PCR, using primers of SEQ ID NO: 1 and 2. Preferably, the levels of IncRNA NEAT1 are measured by PCR, preferably qRT-PCR, using primers of SEQ ID NO: 3 and 4. Preferably, the levels of IncRNA LINC-PINT are measured by PCR, preferably qRT-PCR, using primers of SEQ ID NO: 5 and 6. Preferably, the levels of IncRNA LINC01002 are measured by PCR, preferably qRT-PCR, using primers of SEQ ID NO: 7 and 8. Preferably, the levels of ncRNA PANDAR are measured by PCR, preferably qRT-PCR, using primers of SEQ ID NO: 9 and 10. Preferably, the levels of mRNA TPA (ort-PA) are measured by PCR, preferably qRT-PCR, using primers of SEQ ID NO: 11 and 12. Preferably, the levels of mRNAs IL-1RN are measured by PCR, preferably qRT-PCR, using primers of SEQ ID NO: 13 and 14. Preferably, the levels of mRNA PERP are measured by PCR, preferably qRT-PCR, using primers of SEQ ID NO: 15 and 16. Preferably, the levels of ncRNA U6 are measured by PCR, preferably qRT-PCR, using primers of SEQ ID NO: 17 and 18. See Tale 7 below.

Preferably, the one or more ncRNA are long ncRNA (IncRNA) or micro RNAs (miRNA). The term "IncRNA" encompasses RNA polymerase I (Pol I), Pol II and Pol III transcribed RNAs, and RNAs from processed introns. Long non-coding RNAs be divided into three categories: (1) small RNAs (less than 50 nt); (2) RNA polymerase III (Pol III) transcripts (such as tRNAs, 5S rRNA, 7SK, 7SL, and Alu, vault and Y RNAs37), Pol V transcripts in plants and small Pol II transcripts such as (most) snRNAs and intron-derived snoRNAs38,39 (-50-500 nt); and (3) IncRNAs (more than 500 nt), which are mostly generated by Pol II.

Preferably, step (a) of the method of the present invention comprises measuring the RNA expression levels of at least one or more of the RNAs LINC-PINT, NEAT1, TPA, PERP, and/or IL-1RN, or any combination thereof. Preferably, the one or more RNA measured in (a) are selected from the list consisting of LINC-PINT, NEAT1, TPA, PERP, and IL-1RN, wherein the pregnant female to be screened is at risk of having GDM if the RNA expression levels measured in (a) are increased, preferably statistically significant increased, than the expression levels of the same RNA measured in the control GCF sample. Thus, a preferred embodiment of the first aspect refers to an *in vitro* method for screening or identifying pregnant females at risk of suffering from or developing GDM, based on measuring the expression level of one or more RNAs LINC-PINT, NEAT1, TPA, PERP, and/or IL-1RN, which will be up-regulated in the GCF of pregnant females that are at risk of having said condition. Preferably, the expression levels of RNAs LINC-PINT, NEAT1, TPA, PERP, and/or IL-1RN are measured by PCR, preferably qRT-PCR, preferably using the primers depicted in Table 7. Preferably, the RNAs LINC-PINT, NEAT1, PERP, TPA and/or IL-1RN measured in step (a) have a sequence comprising or consisting of SEQ ID NO: 19-23 (as depicted in Table 8), respectively, or a sequence having at least 50%, 60%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NOs 19-23, respectively.

Preferably, step (a) of the method of the present invention comprises measuring the RNA expression levels of at least one or more of RNAs NEAT1, MALAT1, LINC-PINT, PLAT, PERP, IL-1RN, PANDAR, or any combination thereof. Preferably, the one or more RNA measured in (a) are one or more selected from the list consisting of NEAT1, MALAT1, LINC-PINT, PLAT, PERP, IL-1RN, PANDAR, wherein the pregnant female to be screened is at risk of having GDM if the RNA expression levels measured in (a) are increased, preferably statistically significant increased, than the expression levels of the same RNA measured in the control GCF sample. Thus, a preferred embodiment of the first aspect refers to an *in vitro* method for screening or identifying pregnant females at risk of suffering from or developing GDM, based on measuring the expression level of one or more RNAs NEAT1, MALAT1, LINC-PINT, PLAT, PERP, IL-1RN, PANDAR, which will be up-regulated in the GCF of pregnant females that are at risk of having said condition.

Preferably, step (a) of the method of the present invention comprises measuring the RNA expression levels of at least one or more of RNAs NEAT 1 and LINC-PINT or any combination thereof. Preferably, the one or more RNA measured in (a) are one or more selected from the list consisting of NEAT 1 and LINC-PINT, wherein the pregnant female to be screened is at risk of having GDM if the RNA expression levels measured in (a) are increased, preferably statistically significant increased, than the expression levels of the same RNA measured in the control GCF sample. Thus, a preferred embodiment of the first aspect refers to an *in vitro* method for screening or identifying pregnant females at risk of suffering from or developing GDM, based on measuring the expression level of one or more RNAs selected from NEAT 1 and LINC-PINT, which will be up-regulated in the GCF of pregnant females that are at risk of having said condition.

Preferably, step (a) of the method of the present invention comprises measuring the RNA expression levels of at least one or more of RNAs MALAT1, NEAT1, LINC-PINT, PANDAR, and LINCO01002, or any combination thereof. Preferably, the one or more RNA measured in (a) are one or more selected from the list consisting of MALAT1, NEAT1, LINC-PINT, and PANDAR, wherein the pregnant female to be screened is at risk of having GDM if the RNA expression levels measured in (a) are increased, preferably statistically significant increased, than the expression levels of the same RNA measured in the control GCF sample. Thus, a preferred embodiment of the first aspect refers to an *in vitro* method for screening or identifying pregnant females at risk of suffering from or developing GDM, based on measuring the expression level of one or more RNAs selected from MALAT1, NEAT1, LINC-PINT, and PANDAR, which will be up-regulated in the GCF of pregnant females that are at risk of having said condition.

It is readily evident that the comparison performed in step (b) is performed for the RNAs measured in step (a). That is, if step (a) comprises measuring 5 RNAs selected from Table 1, step (b) is performed by comparing the expression levels of each of those five RNAs versus the expression levels of the same five RNAs obtained in a control GCF sample.

Most preferably, the one or more RNA measured in (a) is a IncRNA selected from the list consisting of NEAT1 and/or LINC-PINT, wherein the pregnant female to be screened is at risk of having GDM if the RNA expression levels measured in (a) are increased, preferably statistically significant increased, than the NEAT1 and/or LINC-PINT, respectively, expression levels measured in the control GCF sample.

In an embodiment, the risk of having GDM is determined in step (b) when the RNA expression levels measured in (a) are:
- at least a base 2 log fold change of ≥1 (indicating upregulation) or ≤-1 (indicating downregulation) than the expression levels of the same RNA measured in control GCF sample, and/or
- statistically significant higher or lower than the expression levels of the same RNA measured in a control GCF sample, wherein the p value is < 0.05.

In an embodiment, the risk of having GDM is determined in step (b) when the one or more RNA expression levels measured in (a) are at least 1.05, 1.1, 1.2, 1.3, 1.4, 1.5, 1.6, 1.7, 1.8, 1.9, 2, 3, 4, 5, 6, 7, 8, 9, 10, 11, 12, 13, 14, 15, 16, 17, 18, 19, 20 times or more or less, depending on the RNA, above or below (up- or down-regulated compared to the expression levels of the same RNAs measured in the control GCF sample.

In an embodiment, the method comprises measuring the RNA expression levels of at least one of the RNAs selected from Table 1 or 6 or 8, in combination with other parameters that give information of the risk of having GDM, such as blood glucose or oral glucose tolerance test (OGTT).

In one embodiment, the differentially expressed RNA(s) measured in step (a) are upregulated compared to the same RNA(s) of a female that did not develop DGM during and/or after pregnancy. In another embodiment the RNA shown in Tables 1, 6 or 8 are upregulated or downregulated in comparison to the same RNA obtained from control GCF sample, preferably wherein the control GCF sample is a female that did not develop GCF during and/or after pregnancy.

In an embodiment, the determination in step (b) of whether the pregnant female screened in (a) is at risk of having GDM is performed by a predictive model which correlates the expression levels of one or more RNA measured in step (a) with representative expression levels of one or more RNA measured in one or more control GCF samples, said predictive model having been generated by training a computer with a plurality of expression levels of one or more RNA from previously identified pregnant female having GDM or not having GDM, by machine learning on said expression levels so as to obtain representative expression levels associated with increased risk of developing GDM.

The term "predictive model" is a mathematical or computational tool used to forecast future outcomes or trends based on historical data and patterns. These models leverage statistical techniques, machine learning algorithms, and data mining methods to identify relationships and trends within data, allowing for the prediction of unknown or future events. The term "predictive model" encompasses a wide range of algorithms and techniques used to forecast future outcomes based on historical data. These algorithms include a wide variety of algorithms, including Bayesian, artificial intelligence, machine learning, deep learning and neural network algorithms, among many others.

In an embodiment, step (b) is performed by a machine learning method selected from a regression method, a classification method or a combination thereof. It will be appreciated that the term "machine learning" generally refers to algorithms that give a computer the ability to learn without being explicitly programmed, including algorithms that learn from and make predictions about data. Machine learning algorithms employed by the embodiments disclosed herein may include, but are not limited to, random forest ("RF"), least absolute shrinkage and selection operator ("LASSO") logistic regression, regularized logistic regression, XGBoost, decision tree learning, artificial neural networks ("ANN"), deep neural networks ("DNN"), support vector machines, rule-based machine learning, and/or others.

For clarity, algorithms such as linear regression or logistic regression can be used as part of a machine learning process. In a particular embodiment, step (b) is performed by a classification method, which results in identifying the pregnant female as a pregnant female at risk of having GDM.

In one embodiment, step (b) is carried out by a classification method; preferably selected from logistic regression, random forest, gradient boosting (GB), adaptive boosting (AB), extreme Gradient Boosting (XGB) k-nearest neighbours (kNN), artificial neural network (ANN), support vector machine (SVM), and combinations thereof.

In an embodiment, the predictive model is generated by training the computer with a plurality of RNA expression profiles from previously identified samples from subjects having GDM, or not having GD, so as to obtain representative multivariable data sets associated with this group of patients; wherein the training comprises the following steps:
(i) training data, from a plurality of RNA expression profiles, is randomly stratified into:
   - a training or calibration dataset (for example in a percentage of about 75%), and
   - a validation dataset (for example in a percentage of about 25%);
(ii) the predictive model is seeded on the calibration dataset (particularly is developed by applying a machine learning method selected from a regression method, a classification method or a combination thereof on the calibration dataset);
(iii) the predictive model is optimized by an internal cross validation; preferably by the leave one out cross validation, wherein the model is trained n times, and each time one variable is left out from the training dataset, which is of *n* size minus one variable; and
(iv) the predictive model is further validated by predicting new samples using the validation or test dataset, which is the variable left out from the training dataset each time; and the overall performance is the average of all individual performances (n).

In some embodiments, step (b) is performed by a classification method wherein the pregnant females screened are assigned a probability of belonging to given category such as pregnant females at risk of developing GDM.

In a particular embodiment, step (b) is performed by a classification method, more in particular, by gradient boosting, which includes the value of one or more variables of the RNA expression profiles collected in step (i) and which contribute to the identification of pregnant females at risk of developing GDM.

In an embodiment, the woman to be screeded whose RNA expression levels or profile are measured in step (a) has not developed or does not have GDM. Preferably, the pregnant female to be screened in step (a) is a pregnant woman that is in the first trimester of pregnancy. Preferably, the pregnant female to be screened in step (a) is in week 1-14, preferably week 7-14, more preferably week 11-14, of pregnancy.

If it is determined that the pregnant female that has been screened using the method of the present invention has an increased risk of developing GDM, the attending health care provider may subsequently prescribe or institute a treatment program. In this manner, the present invention also provides for methods of screening individuals as candidates for treatment of GDM.

Thus, the method of the present invention may comprise a further step (c) comprising treating the pregnant female screened if step (b) determined that she is at risk of developing gestational diabetes mellitus. Preferably, the treatment applied to said pregnant female in order to avoid, reduce, or ameliorate the symptoms or the development of GDM. Preferably, the treatment includes administering the subject insulin - normally metformin.

Hence, in an embodiment, the present invention refers to a method of treating or preventing GDM in a subject that has been diagnosed as being at risk of having GDM, the method comprising:
(a) measuring the nucleic acid expression levels of one or more RNAs in a gingival crevicular fluid (GCF) sample obtained from a pregnant female to be screened, as defined above,
(b) comparing the RNA expression levels obtained in (a) with the expression levels of the same one or more RNA measured in a control GCF sample and determining that the pregnant female screened in (a) is at risk of having gestational diabetes mellitus if the RNA expression levels measured in (a) are deviated or different compared to the RNA expression levels measured in the control GCF sample, as defined above, and
(c) treating the pregnant female screened if step (b) determined that she is at risk of developing gestational diabetes mellitus.

Similarly, the invention provides methods of monitoring the effectiveness of a treatment for GDM. Once a treatment regimen has been established, with or without the use of the methods of the present invention to assist in a diagnosis of GDM, the methods of monitoring a pregnant female's RNA expression levels over time can be used to assess the effectiveness of a GDM treatment. Specifically pregnant female's RNA expression levels can be assessed over time, including before, during and after treatments for GDM.

### The following embodiments are considered preferred:

In an embodiment, the method for screening or identifying pregnant females at risk of developing gestational diabetes mellitus (GDM) comprises the steps of:
(a) measuring the nucleic acid expression levels of one or more RNAs in a gingival crevicular fluid (GCF) sample obtained from a pregnant female to be screened, wherein the RNAs measured are one or more selected from the list of Table 1, and
(b) comparing the RNA expression levels obtained in (a) with the expression levels of the same one or more RNA measured in a control GCF sample and determining that the pregnant female screened in (a) is at risk of having gestational diabetes mellitus if the RNA expression levels measured in (a) are deviated or differentially expressed compared to the RNA expression levels measured in the control GCF sample.

In an embodiment, the method for screening or identifying pregnant females at risk of developing gestational diabetes mellitus (GDM) comprises the steps of:
(a) measuring the nucleic acid expression levels of one or more RNAs in a gingival crevicular fluid (GCF) sample obtained from a pregnant female to be screened, wherein the RNAs measured are one or more selected from the list of Table 6, and
(b) comparing the RNA expression levels obtained in (a) with the expression levels of the same one or more RNA measured in a control GCF sample and determining that the pregnant female screened in (a) is at risk of having gestational diabetes mellitus if the RNA expression levels measured in (a) are deviated or differentially expressed compared to the RNA expression levels measured in the control GCF sample.

In an embodiment, the method for screening or identifying pregnant females at risk of developing gestational diabetes mellitus (GDM) comprises the steps of:
(a) measuring the nucleic acid expression levels of one or more RNAs in a gingival crevicular fluid (GCF) sample obtained from a pregnant female to be screened, wherein the RNAs measured are one or more selected from the list of Table 8, and
(b) comparing the RNA expression levels obtained in (a) with the expression levels of the same one or more RNA measured in a control GCF sample and determining that the pregnant female screened in (a) is at risk of having gestational diabetes mellitus if the RNA expression levels measured in (a) are deviated or differentially expressed compared to the RNA expression levels measured in the control GCF sample.

In an embodiment, the method for screening or identifying pregnant females at risk of developing gestational diabetes mellitus (GDM) comprises the steps of:
(a) measuring the nucleic acid expression levels of one or more RNAs in a gingival crevicular fluid (GCF) sample obtained from a pregnant female to be screened, wherein the RNAs measured are one or more selected from the list consisting of NEAT1, LINC00673, MIR205HG, MALAT1, PP7080, RUNX1-IT1, LINC-PINT, LINC00342, LRRC75A-AS1, PSMA3-AS1, RDH10-AS1, PANDAR, and any combination thereof, and
(b) comparing the RNA expression levels obtained in (a) with the expression levels of the same one or more RNA measured in a control GCF sample and determining that the pregnant female screened in (a) is at risk of having gestational diabetes mellitus if the RNA expression levels measured in (a) are increased, preferably statistically significant increased, compared to the RNA expression levels measured in the control GCF sample.

In an embodiment, the method for screening or identifying pregnant females at risk of developing gestational diabetes mellitus (GDM) comprises the steps of:
(a) measuring the nucleic acid expression levels of one or more RNAs in a gingival crevicular fluid (GCF) sample obtained from a pregnant female to be screened, wherein the RNAs measured are one or more selected from the list consisting of NEAT1, MALAT1, LINC-PINT, PLAT, PERP, IL1RN, PANDAR, and any combination thereof, and
(b) comparing the RNA expression levels obtained in (a) with the expression levels of the same one or more RNA measured in a control GCF sample and determining that the pregnant female screened in (a) is at risk of having gestational diabetes mellitus if the RNA expression levels measured in (a) are increased, preferably statistically significant increased, compared to the RNA expression levels measured in the control GCF sample.

In an embodiment, the method for screening or identifying pregnant females at risk of developing gestational diabetes mellitus (GDM) comprises the steps of:
(a) measuring the nucleic acid expression levels of one or more RNAs in a gingival crevicular fluid (GCF) sample obtained from a pregnant female to be screened, wherein the RNAs measured are one or more selected from the list consisting of LINC-PINT, NEAT1, TPA, PERP, IL-1RN and any combination thereof, and
(b) comparing the RNA expression levels obtained in (a) with the expression levels of the same one or more RNA measured in a control GCF sample and determining that the pregnant female screened in (a) is at risk of having gestational diabetes mellitus if the RNA expression levels measured in (a) are increased, preferably statistically significant increased, compared to the RNA expression levels measured in the control GCF sample.

In an embodiment, the method for screening or identifying pregnant females at risk of developing gestational diabetes mellitus (GDM) comprises the steps of:
(a) measuring the nucleic acid expression levels of one or more RNAs in a gingival crevicular fluid (GCF) sample obtained from a pregnant female to be screened, wherein the RNAs measured are one or more selected from the list consisting of NEAT1, LINC-PINT or any combination thereof, and
(b) comparing the RNA expression levels obtained in (a) with the expression levels of the same one or more RNA measured in a control GCF sample and determining that the pregnant female screened in (a) is at risk of having gestational diabetes mellitus if the RNA expression levels measured in (a) are increased, preferably statistically significant increased, compared to the RNA expression levels measured in the control GCF sample.

In an embodiment, the method for screening or identifying pregnant females at risk of developing gestational diabetes mellitus (GDM) comprises the steps of:
(a) measuring the nucleic acid expression levels of one or more RNAs in a gingival crevicular fluid (GCF) sample obtained from a pregnant female to be screened, wherein the RNAs measured are one or more selected from the list consisting of MALAT1, NEAT1, LINC-PINT, PANDAR, or any combination thereof, and
(b) comparing the RNA expression levels obtained in (a) with the expression levels of the same one or more RNA measured in a control GCF sample and determining that the pregnant female screened in (a) is at risk of having gestational diabetes mellitus if the RNA expression levels measured in (a) are increased, preferably statistically significant increased, compared to the RNA expression levels measured in the control GCF sample.

In an embodiment, the method for screening or identifying pregnant females at risk of developing gestational diabetes mellitus (GDM) comprises the steps of:
(a) measuring the nucleic acid expression levels of one or more RNAs in a gingival crevicular fluid (GCF) sample obtained from a pregnant female to be screened, wherein the RNAs measured are selected from the list consisting of LINC01166, LOC285696, SUCLG2-AS1, C15orf32, C9orf135-AS1, LINC00960, FLJ33534, LINC01604, C2orf91, FLJ16779, MORC2-AS1, LINC01002, C7orf65, CTBP1-AS, TMEM105, EPHA1-AS1, SOCS2-AS1, VPS9D1-AS1, C9orf139, LOC729737, EMX2OS, LINC01314, RRN3P2, LOC101928093, WDFY3-AS2, LOC221946, LOC101929567, LOC100506022, LOC100288152, MAP3K14, LOC100129697, LINC00324 and any combination thereof, and
(b) comparing the RNA expression levels obtained in (a) with the expression levels of the same one or more RNA measured in a control GCF sample and determining that the pregnant female screened in (a) is at risk of having gestational diabetes mellitus if the RNA expression levels measured in (a) are decreased, preferably statistically significant decreased, compared to the RNA expression levels measured in the control GCF sample.

In a second aspect, the present invention refers to a predictive or prognosis method. In particular, to an *in vitro* predictive method for determining the risk of developing GDM in a pregnant female, the method comprising the steps as defined in the first aspect or any of its embodiments, preferably the steps of:
(a) measuring expression levels of one or more RNAs in a GCF sample obtained from a pregnant female to be screened, and
(b) comparing the RNA expression levels obtained in (a) with the expression levels of the same one or more RNA measured in a control GCF sample, and determining that the pregnant female screened in (a) is at risk of having GDM if the RNA expression levels measured in (a) are deviated or differentially expressed compared to the RNA expression levels measured the control GCF sample.

In **a third aspect,** the invention relates to an *in vitro* method for selecting a pregnant female at risk of developing GDM as a candidate to receive an adequate therapy to treat said GDM, the method comprising the steps as defined in the first aspect or any of its embodiments, preferably the steps of:
(a) measuring expression levels of one or more RNAs in a GCF sample obtained from a pregnant female to be screened, and
(b) comparing the RNA expression levels obtained in (a) with the expression levels of the same one or more RNA measured in a control GCF sample, and determining that the pregnant female screened in (a) is at risk of having GDM and thus it is a candidate to receive therapy to treat GDM if the RNA expression levels measured in (a) are deviated or differentially expressed compared to the RNA expression levels measured the control GCF sample.

In **a fourth aspect,** the invention also provides *in vitro* methods for the treatment of pregnant females that have been identified as at risk of having GDM by any of the methods according to the invention, wherein if the pregnant female has been identified as a patient at risk of GDM, she is treated with a therapy adequate for the treatment of GMD.

The term "treatment", as used herein comprises any type of therapy, which aims at terminating, preventing, ameliorating and/or reducing the susceptibility to a clinical condition as described herein. In a preferred embodiment, the term treatment relates to prophylactic treatment (i.e. a therapy to reduce the susceptibility of a clinical condition, a disorder or condition as defined herein). Thus, "treatment," "treating," and the like, as used herein, refer to obtaining a desired pharmacologic and/or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, and/or immune deficiency.

Preferably, the treatment given to the pregnant female is appropriate diabetic diet or food plan and/or the administration of oral hypoglycemic drugs such as metformin.

In **a fifth aspect,** the present invention refers to an *in vitro* method for monitoring the response to a therapy or for monitoring the progression of GDM in a pregnant female, the method comprising the steps as defined in the first aspect or in any of its embodiments, preferably the steps of:
(a) measuring expression levels of one or more RNAs in a GCF sample obtained from a pregnant female to be screened, and
(b) comparing the RNA expression levels obtained in (a) with the expression levels of the same one or more RNA measured in a control GCF sample, and determining that the pregnant female screened in (a) is progressing towards hacing GDM if the RNA expression levels measured in (a) are deviated or differentially expressed compared to the RNA expression levels measured the control GCF sample.

In **a sixth aspect,** the present invention refers to an *in vitro* method for the diagnosis, in particular the early diagnosis, of GDM in a pregnant female, the method comprising step (a) as defined in the first aspect or in any of its embodiments, and a step (b) of diagnosing GDM if the RNA expression levels measured in (a) are deviated or differentially expressed compared to the expression levels of the same RNA measured in a control GCF sample. This aspect also includes a method of treating a pregnant female that has been diagnosed with GDM, or that is at risk of having GDM.

In **a seventh aspect,** the invention relates to a kit, package or device that contains reagents adequate for implementing any of the methods of the previous aspects (methods of the invention). It will be understood that, depending on the nature of the method, the reagents adequate for its implementation will vary.

In the context of the present invention, "kit" is understood as a product containing the different reagents required for carrying out the methods of the invention packaged such that it allows being transported and stored. The materials suitable for the packaging of the components of the kit include glass, plastic (polyethylene, polypropylene, polycarbonate, and the like), bottles, vials, paper, sachets, and the like. Where there are more than one component in a kit they may be packaged together if suitable or the kit will generally contain a second, third or other additional container into which the additional components may be separately placed. However, in some embodiments, certain combinations of components may be packaged together comprised in one container means. A kit can also include a means for containing any reagent containers in close confinement for commercial sale. Such containers may include injection or blow-molded plastic containers into which the desired vials are retained. One or more compositions of a kit can be lyophilized. In some embodiments, all compositions of a kit of the disclosure will be lyophilized. In some embodiments, a kit of the disclosure with one or more lyophilized agents will be supplied with a re-constitution buffer. Reagents and components of kits may be comprised in one or more suitable container means. A container means may generally comprise at least one vial, test tube, flask, bottle, syringe or other container means, into which a component may be placed, and preferably, suitably aliquoted.

Furthermore, the kits of the invention can contain instructions for the simultaneous, sequential, or separate use of the different components that are in the kit. Said instructions can be in the form of printed material or in the form of an electronic medium capable of storing instructions such that they can be read by a subject, such as electronic storage media (magnetic disks, tapes, and the like), optical media (CD-ROM, DVD), and the like. The media may additionally or alternatively contain Internet addresses providing said instructions.

Preferably, the kit comprises one or more biomarker detecting reagents for measuring in a gingival crevicular fluid (GCF) sample obtained from a pregnant female the RNA expression levels of one or more RNAs selected from Table 1 or 6 or 8, preferably selected from the list consisting of LINC-PINT, NEAT1, TPA, PERP, and IL-1RN.

The sixth aspect of the invention further refers to uses of said kit for screening for pregnant females at risk of developing GDM.

In **an eighth aspect,** the invention refers to a method for obtaining useful data for the in vitro diagnosis or prognosis of GDM, the method comprising the steps as defined in the first aspect of the invention, or any of its embodiments.

It is noted that any of the methods described in the present invention can be performed using software, hardware, firmware, hardwiring, or combinations of any of these. Accordingly, in **a ninth aspect,** the invention relates to a computer-implemented method, wherein the method is any of the methods according to the invention. Preferably, the invention relates to a computer containing instructions for carrying any of the methods described herein.

In one embodiment, the invention relates to a computer implemented method for determining whether a pregnant female is at risk of having GDM, the method comprising the steps as defined in the first aspect of the invention, or any of its embodiments. In another embodiment, the invention relates to a computer implemented method for classifying a pregnant female at risk of having GDM, the steps as defined in the first aspect of the invention, or any of its embodiments.

It is further noted that preferably, for any of the methods according to the invention the pregnant female is a pregnant woman, preferably a pregnant woman that is the first trimester of pregnancy, preferably in 11-14 weeks of pregnancy. Also, preferably for any of the methods described herein, the RNAs are selected from Table 1, preferably selected from the list consisting of LING-PINT, NEAT1, TPA, PERP, and IL-1RN, most preferably NEAT1 and/or LINC-PINT.

The invention is described below by way of the following examples, which are merely illustrative and do not limit the scope of the invention.

The invention is described below by the following examples, which must be considered as merely illustrative and in no case limiting of the scope of the present invention.

### EXAMPLES

### Methods

### Study Design

This was a prospective cohort study. The cohort comprised 257 pregnant women enrolled at 11-14 weeks of gestation. Each pregnant woman received prenatal care and was followed from enrollment until delivery. Demographic, clinical, physical, obstetric, and medical history were recorded. A specialist in periodontics performed a complete periodontal evaluation, including periodontal probing depth (PPD), clinical attachment level (CAL), bleeding on probing (BOP), plaque index (PI), (visible plaque accumulation), measured along the gingival margin and recorded as presence (+) or absence (-).

Pregnant women were enrolled in Clinica Davila (Santiago, Chile). The diagnosis of Gestational Diabetes mellitus (GDM) was made using an oral glucose tolerance test (OGTT) at 24-28 weeks of gestation and was used as the primary outcome variable. Participants diagnosed with GDM were assigned to the GDM group. Otherwise, they were assigned to the non-GDM group. Pregnant women were included if they were between 18 and 40 years of age, had an 11-14-week pregnancy, and had a prior pregnancy diagnosis confirmed by a transvaginal ultrasound scan to confirm gestational age, number of embryos, and viability, with or without a diagnosis of periodontal disease. Women were excluded if they had type 1 or 2 diabetes, fewer than 18 teeth, any history of periodontal therapy during the last six months, or systemic or topical antimicrobial/anti-inflammatory therapy in the previous three months. Written informed consent was obtained from women who agreed to participate in the study, which was approved by the Clinic Davila Ethics Committee and conducted in accordance with the Declaration of 1973, as revised in 2003. The study protocol was explained to all the participants who provided informed consent.

The variables studied were GDM diagnosis, periodontal diagnosis, fasting first- and second-trimester blood glucose concentrations, age, first-trimester body mass index (BMI), smoking during pregnancy, pre-pregnancy smoking history, and RT-qPCR expression of MALAT1, NEAT1, LINC-PINT, PANDAR, LINC011002, PLAT, PERP, and IL-1RN in gingival crevicular fluid samples at 11-14 weeks of pregnancy.

### Definitions

Gestational diabetes mellitus was diagnosed at 24-28 weeks of gestation using the following parameters: a criterion of diagnosis of GDM was made by 75 g oral glucose tolerance testing (OGTT) and 2 hour 8.5 mmol/l by universal glucose tolerance testing (fasting plasma glucose concentration of ≥ 93 mmol/l and a 2h blood glucose concentration of ≥ 153 mmol/I 2h after oral administration of 75 g glucose according to the IADPSG criteria). Periodontitis was defined as stated by the 2017 World Workshop as interdental CAL detectable in ≥ 2 non-adjacent teeth, or buccal or oral CAL ≥ 3 mm with pocketing > 3 mm detectable in ≥ 2 teeth. Gingivitis was defined as subjects who did not exhibit PPD greater or equal to 3 mm, without CAL and positive BOP in ≥ 10%. Gingival health was defined as < 10% bleeding at probing sites with PPD ≤ 3 mm. Periodontitis stages I and II were determined based on the CAL levels. When CAL was between 1-2 mm, the patient was diagnosed with stage I periodontitis. When CAL was between 3-4 mm, the patient was diagnosed with stage II periodontitis. When CAL was 5 mm or more, with four teeth or less missing, and in the presence of ten or more occluding pairs, in the absence of bite collapse, drifting, flaring, or a severe ridge defect, the diagnosis was stage III periodontitis. In the presence of CAL > 5 mm, with four or more missing teeth, absence of 10 occluding pairs, or bite collapse, drifting, flaring, or a severe ridge defect, the diagnosis was Periodontitis Stage IV. Radiographic bone loss was not assessed because of pregnancy. Therefore, the periodontitis grade has not yet been established.

### Gingival crevicular fluid (GCF) collection and elution protocol

GCF samples were obtained from four periodontal pockets and/or sites depending on periodontal diagnosis (most affected periodontal site/pocket × quadrant) at the same gestational age period in all pregnancies (11-14 weeks of gestation). The paper strips* were placed into the sulci/pocket until mild resistance was sensed, left in place for 30s and pooled. Strips contaminated with saliva or blood were excluded from this study.

### GCF RNA extraction and library construction

Total RNA from GCF samples was isolated. The RNA concentration was measured using a Qubit 4.0 fluorometer, and its integrity was determined using the Fragment Analyzer^{™} Automated CE System (Table 5). DNA libraries were constructed using 500 ng of total RNA from each sample using the Collibri^{™} Stranded RNA Library Prep Kit (Thermo Fisher) for Illumina Systems following the manufacturer's instructions. Fragment Analyzer^{™} was used to verify library sizes. Sequencing was performed on a HiSeq platform (Illumina).

### RNA-Seq Analysis

Reads were aligned to the human reference genome (GRCh38) using Bowtie2 software (Table 5). HTseq software was used to determine the gene abundance and calculate the raw read number for each gene. Differentially expressed genes (DEGs) were estimated using DESeq2 in the SARTools R package. The RNA candidates were selected based on genes meeting the criteria of having a minimum of three reads in at least three replicates for each group (control or GDM), exhibiting a log2 fold change of ≥1 (indicating upregulation) or ≤-1 (indicating downregulation), with p-values < 0.05.

### RT-qPCR non-coding RNA validation

The non-coding RNAs candidates were validated by Reverse Transcription Quantitative Polymerase Chain Reaction (RT-qPCR) in the GCF samples of the complete cohort of pregnant women (Table 6) (250 control and 25 GDM patients). Targeted validation confirmed the expression of genes associated with GDM. For the sequence of the primers, see table 7 below.

### Statistical Analysis

The normality of continuous quantitative variables was assessed using the Shapiro-Wilk test. They were described through the median and the 25^{th} and 75^{th} percentiles. Differences between the GDM and normal groups were evaluated using the Mann-Whitney U test. Categorical variables were described as absolute and percentage frequencies and were evaluated using Pearson's chi-square test. The association strength was assessed using a multiple logistic regression model. The diagnostic accuracy of IncRNAs in GCF samples was evaluated by constructing operating characteristic curves and calculating the area under the curve (AUC-ROC). The optimal cut-off points for estimating Youden's index showed the highest sensitivity and specificity. The goodness of fit and internal validation of the model were assessed using bootstrapping. Data management and statistical analyses were performed using the Stata software 2015. Stata Statistical Software: Release 14.1 (StataCorp LP, College Station, TX, USA). Statistical significance was set at p < 0.05.

### RESULTS

In total, 257 pregnant women were enrolled in this prospective cohort study. Of them, 9,3% (24/257) were diagnosed with GDM. The baseline characteristics of the study population are summarized in Table 3. Women who developed GDM showed statistically significant differences in first trimester fasting glucose concentrations (p < 0.001), age (p = 0.03), and initial body mass index (p = 0.02) at 11-14 weeks of pregnancy. Regarding periodontal diagnosis, 36.6% of pregnant women were diagnosed as gingival healthy or mild gingivitis and 63.4% had periodontitis.

Reads were obtained from sequencing that were aligned to the human reference genome (GRCh38) using Bowtie2 software. Gene abundance and raw read counts for each gene were determined using the Htseq software. Differentially expressed genes (DEGs) were identified using DESeq2 in the SARTools R package. IncRNA and miRNA candidates were selected based on the following criteria: a minimum of three reads in at least three replicates per group (control or GDM), a log2 fold change of ≥1 (upregulation) or ≤-1 (downregulation), and p < 0.05.

**TABLE 2**

| Id | GeneType | CTL | GDM | FoldChange | log2FoldChange | pvalue |
|---|---|---|---|---|---|---|
| LINC01166 | IncRNA | 11 | 0 | 0.012 | -6.322 | 1.93E-06 |
| SUCLG2-AS1 | IncRNA | 5 | 0 | 0.02 | -5.669 | 0.00036 709 |
| C9orf135-AS1 | lncRNA | 4 | 0 | 0.02 | -5.613 | 0.00137 597 |
| FLJ33534 | IncRNA | 3 | 0 | 0.024 | -5.366 | 0.00130 63 |
| C2orf91 | lncRNA | 3 | 0 | 0.026 | -5.242 | 0.00468 644 |
| MORC2-AS1 | IncRNA | 4 | 0 | 0.044 | -4.504 | 0.00401 303 |
| C7orf65 | IncRNA | 5 | 0 | 0.045 | -4.488 | 0.00216 1 |
| TMEM105 | IncRNA | 6 | 0 | 0.052 | -4.262 | 0.00425 26 |
| SOCS2-AS1 | IncRNA | 3 | 0 | 0.061 | -4.023 | 0.01709 313 |
| C9orf139 | IncRNA | 6 | 0 | 0.068 | -3.877 | 0.00447 185 |
| EMX2OS | IncRNA | 11 | 1 | 0.069 | -3.854 | 0.00022 552 |
| RRN3P2 | IncRNA | 11 | 1 | 0.069 | -3.85 | 0.00061 594 |
| WDFY3-AS2 | IncRNA | 3 | 0 | 0.07 | -3.827 | 0.02365 429 |
| LOC10192 9567 | IncRNA | 6 | 1 | 0.097 | -3.364 | 0.01220 136 |
| LOC10028 8152 | IncRNA | 4 | 1 | 0.108 | -3.205 | 0.01287 462 |
| LOC10012 9697 | IncRNA | 7 | 1 | 0.115 | -3.117 | 0.00370 291 |
| LOC28569 6 | IncRNA | 6 | 1 | 0.121 | -3.045 | 0.02417 31 |
| C15orf32 | IncRNA | 6 | 1 | 0.132 | -2.92 | 0.01747 36 |
| LINC00960 | IncRNA | 11 | 2 | 0.139 | -2.848 | 0.02781 478 |
| LINC01604 | IncRNA | 56 | 8 | 0.146 | -2.778 | 0.00047 102 |
| FLJ16779 | IncRNA | 14 | 2 | 0.148 | -2.752 | 0.01727 119 |
| LINC01002 | IncRNA | 496 | 88 | 0.178 | -2.494 | 0.00609 499 |
| CTBP1-AS | IncRNA | 10 | 2 | 0.183 | -2.45 | 0.04297 725 |
| EPHA1-AS1 | IncRNA | 8 | 2 | 0.22 | -2.182 | 0.02426 725 |
| VPS9D1-AS1 | IncRNA | 34 | 8 | 0.221 | -2.18 | 0.00544 276 |
| LOC72973 7 | IncRNA | 302 | 74 | 0.245 | -2.028 | 0.01312 952 |
| LINC01314 | IncRNA | 10 | 2 | 0.248 | -2.014 | 0.03698 939 |
| LOC10192 8093 | IncRNA | 34 | 9 | 0.257 | -1.961 | 0.03853 233 |
| LOC22194 6 | IncRNA | 21 | 6 | 0.261 | -1.936 | 0.03091 862 |
| LOC10050 6022 | IncRNA | 22 | 6 | 0.277 | -1.853 | 0.04926 999 |
| MAP3K14 | IncRNA | 42 | 14 | 0.32 | -1.644 | 0.04321 717 |
| LINC00324 | IncRNA | 111 | 41 | 0.365 | -1.453 | 0.03732 566 |
| N EAT1 | IncRNA | 1335 | 3867 | 2.895 | 1.534 | 0.00500 089 |
| LINC00673 | IncRNA | 3 | 10 | 4.044 | 2.016 | 0.03849 672 |
| MIR205HG | lncRNA | 2 | 12 | 4.55 | 2.186 | 0.03497 605 |
| MALAT1 | IncRNA | 4470 | 2072 8 | 4.637 | 2.213 | 0.00046 386 |
| PP7080 | IncRNA | 2 | 11 | 4.767 | 2.253 | 0.03029 804 |
| RU NX1-IT1 | lncRNA | 1 | 8 | 6.32 | 2.66 | 0.02543 237 |
| LINC-PINT | IncRNA | 5 | 37 | 6.58 | 2.718 | 0.00033 423 |
| LINC00342 | lncRNA | 4 | 25 | 7.124 | 2.833 | 0.00148 802 |
| LRRC75A-AS1 | IncRNA | 1 | 7 | 8.959 | 3.163 | 0.01655 028 |
| PSMA3-AS1 | IncRNA | 2 | 11 | 10.415 | 3.381 | 0.00138 377 |
| RDH10-AS1 | lncRNA | 2 | 25 | 13.707 | 3.777 | 2.25E-05 |
| PANDAR | IncRNA | 0 | 9 | 28.139 | 4.814 | 0.00098 053 |
| MIR16-2 | miRNA | 8 | 0 | 0.035 | -4.82 | 0.00042 403 |
| MIR199B | miRNA | 19 | 1 | 0.044 | -4.513 | 2.06E-05 |
| MIR339 | miRNA | 9 | 1 | 0.048 | -4.379 | 0.00046 02 |
| MIR185 | miRNA | 44 | 2 | 0.053 | -4.243 | 1.48E-06 |
| MIR16-1 | miRNA | 18 | 1 | 0.054 | -4.219 | 2.45E-06 |
| MIR454 | miRNA | 11 | 1 | 0.061 | -4.045 | 0.00117 979 |
| MIR25 | miRNA | 53 | 3 | 0.061 | -4.034 | 1.73E-06 |
| MIR183 | miRNA | 7 | 0 | 0.062 | -4.002 | 0.00065 928 |
| MIR766 | miRNA | 12 | 1 | 0.064 | -3.968 | 5.50E-05 |
| MIR1249 | miRNA | 18 | 1 | 0.066 | -3.923 | 2.78E-05 |
| MIR652 | miRNA | 44 | 3 | 0.066 | -3.915 | 1.26E-06 |
| MIR148A | miRNA | 39 | 3 | 0.068 | -3.882 | 6.73E-06 |
| MIR15A | miRNA | 42 | 3 | 0.068 | -3.881 | 0.00047 316 |
| MIR140 | miRNA | 172 | 13 | 0.072 | -3.787 | 1.39E-07 |
| MIR145 | miRNA | 297 | 22 | 0.074 | -3.762 | 3.51E-09 |
| MIR505 | miRNA | 6 | 1 | 0.075 | -3.731 | 0.00263 975 |
| MIR425 | miRNA | 152 | 12 | 0.076 | -3.711 | 3.40E-09 |
| MIR424 | miRNA | 28 | 2 | 0.078 | -3.672 | 2.48E-05 |
| MIR223 | miRNA | 20107 | 1677 | 0.083 | -3.584 | 4.32E-11 |
| MIR345 | miRNA | 12 | 1 | 0.084 | -3.576 | 0.00092 299 |
| MIR93 | miRNA | 210 | 19 | 0.089 | -3.496 | 1.31E-07 |
| MIR6821 | miRNA | 12 | 1 | 0.089 | -3.495 | 0.00106 401 |
| MIR361 | miRNA | 16 | 2 | 0.09 | -3.481 | 0.00022 422 |
| MIR590 | miRNA | 23 | 2 | 0.091 | -3.451 | 0.00069 574 |
| MIR7-1 | miRNA | 9 | 1 | 0.093 | -3.433 | 0.00060 406 |
| MIR106B | miRNA | 7 | 1 | 0.094 | -3.412 | 0.00227 234 |
| MIR30E | miRNA | 75 | 7 | 0.097 | -3.371 | 3.45E-08 |
| MIR187 | miRNA | 5 | 1 | 0.098 | -3.353 | 0.02772 038 |
| MIR193A | miRNA | 20 | 2 | 0.102 | -3.289 | 6.20E-05 |
| MIR15B | miRNA | 93 | 10 | 0.102 | -3.286 | 1.09E-06 |
| MIR186 | miRNA | 47 | 5 | 0.103 | -3.282 | 2.13E-06 |
| M I R34A | miRNA | 7 | 1 | 0.103 | -3.279 | 0.00220 128 |
| MIR130A | miRNA | 9 | 1 | 0.124 | -3.008 | 0.03592 715 |
| MIR146A | miRNA | 15 | 2 | 0.126 | -2.988 | 0.00488 094 |
| MIR8055 | miRNA | 7 | 1 | 0.138 | -2.862 | 0.03437 821 |
| MIR197 | miRNA | 362 | 50 | 0.138 | -2.859 | 3.25E-09 |
| MIR628 | miRNA | 10 | 2 | 0.141 | -2.828 | 0.02245 748 |
| MIR142 | miRNA | 102 | 15 | 0.142 | -2.818 | 8.99E-06 |
| MIR324 | miRNA | 18 | 3 | 0.147 | -2.764 | 0.00907 112 |
| MIR26B | miRNA | 63 | 10 | 0.152 | -2.72 | 3.29E-06 |
| MIR23A | miRNA | 1349 | 209 | 0.154 | -2.697 | 4.31E-06 |
| MIR421 | miRNA | 13 | 2 | 0.155 | -2.689 | 0.00167 374 |
| MIR132 | miRNA | 7 | 1 | 0.158 | -2.666 | 0.01945 395 |
| MIR191 | miRNA | 1462 | 236 | 0.161 | -2.634 | 1.21E-06 |
| MIR96 | miRNA | 12 | 2 | 0.166 | -2.589 | 0.01144 674 |
| MIR3613 | miRNA | 73 | 13 | 0.176 | -2.508 | 0.00044 408 |
| MIR30D | miRNA | 86 | 16 | 0.177 | -2.5 | 0.00030 762 |
| MIR328 | miRNA | 8 | 2 | 0.188 | -2.413 | 0.04875 704 |
| MIR30B | miRNA | 17 | 4 | 0.198 | -2.334 | 0.01109 494 |
| MIR532 | miRNA | 19 | 5 | 0.218 | -2.2 | 0.01196 705 |
| MIR320A | miRNA | 329 | 73 | 0.22 | -2.182 | 0.00013 67 |
| MIRLET7D | miRNA | 62 | 14 | 0.222 | -2.171 | 0.00096 113 |
| MIRLET7I | miRNA | 50 | 12 | 0.225 | -2.151 | 0.00166 856 |
| MIR21 | miRNA | 1113 | 260 | 0.233 | -2.103 | 0.00126 366 |
| M I R4449 | miRNA | 7887 | 1886 | 0.239 | -2.064 | 0.00082 347 |
| MIR221 | miRNA | 73 | 19 | 0.252 | -1.989 | 0.00216 639 |
| MIR29A | miRNA | 28 | 7 | 0.257 | -1.96 | 0.00348 984 |
| MIRLET7G | miRNA | 68 | 18 | 0.262 | -1.935 | 0.00526 179 |
| MIR708 | miRNA | 39 | 11 | 0.273 | -1.871 | 0.01475 143 |
| MIR200C | miRNA | 114 | 32 | 0.274 | -1.87 | 0.00538 139 |
| MIR222 | miRNA | 45 | 13 | 0.278 | -1.844 | 0.00791 475 |
| MIR342 | miRNA | 46 | 13 | 0.281 | -1.833 | 0.04119 213 |
| MIR205 | miRNA | 531 | 151 | 0.283 | -1.821 | 0.00272 231 |
| MIR210 | miRNA | 12 | 3 | 0.292 | -1.777 | 0.03487 002 |
| MIR193B | miRNA | 34 | 11 | 0.303 | -1.721 | 0.03345 226 |
| MIR574 | miRNA | 63 | 21 | 0.319 | -1.647 | 0.00813 672 |
| MIR99A | miRNA | 36 | 13 | 0.354 | -1.5 | 0.04232 958 |
| MIR27A | miRNA | 59 | 24 | 0.399 | -1.324 | 0.00721 953 |
| MIR5047 | miRNA | 4 | 13 | 3.451 | 1.787 | 0.04392 079 |

### Differentially Expressed Genes' Identification in GDM Samples

A total of 26,485 genes were analyzed in the normal and GDM groups (Figure 1A). Of these genes, only 15% were identified as long non-coding RNAs (IncRNAs) (Figure 1B). The data revealed a significant number of genes that were either upregulated (2750) or downregulated (2072), as shown in Figure 1C. Among these genes, 12 IncRNAs were upregulated and 32 IncRNAs were downregulated (Figure 1D). Only five IncRNAs (MALAT1, NEAT1, LINC-PINT, PANDAR, and LINC01002) were selected for analysis because of their specific expression profiles (Figure 2), which could be identified by RT-qPCR in the gingival crevicular fluid (GCF) (Figure 3).

In our analysis, certain coding genes that exhibited dysregulation in diabetes served as controls (Figure 2 and 3). These included tissue-type plasminogen activator (PLAT or t-PA), P53 apoptosis effector related to PMP22 (PERP) and IL-1 receptor antagonist (IL1RN). Our results indicate that the expression patterns of our long non-coding RNA candidates follow a similar trend to that observed in our bioinformatic analysis, which highlights the IncRNAs NEAT1 and LINC-PINT. These IncRNAs showed statistically significant differences in expression levels between the control and gestational diabetes mellitus (GDM) groups in both bioinformatics (figure 2) and RT-qPCR validation (Figure 3) experiments. In contrast, our control genes exhibited a comparable trend between their expression profiles and the validation experiments.

The multivariate regression model identified an association between the quantitative expression of TPA (OR: 0.47; p-value = 0.008; Cl 95% 0.27 - 0.82), PERP (OR: 2.32; p-value = 0.010; Cl 95% 1.22 - 4.40), NEAT (OR: 0.54; p-value = 0.089; Cl 95% 0.26 - 1.10), LINC-PINT (OR: 1.86; p-value = 0.048; Cl 95% 1.01-3.43), IL-1RN (OR: 1.69; p-value = 0.087; Cl 95% 0.93 - 3.10) in GCF, during the first trimester of pregnancy, combined with the initial body mass index (OR: 1.13; p-value = 0.028; Cl 95% 1.01 - 1.26) and first trimester fasting glycemia (OR: 1.21; p-value <0.001; Cl 95% 1.11 - 1.33), with the development of GDM (Table 4A). The internal validation with bootstrapping was like that observed in the multivariate logistic regression model. The model was found to be a good predictor of GDM, with an ROC-AUC of 84% (95% Cl: 0.737-0.931) (Figure 2), specificity of 78.1%, sensitivity of 70.8%, PPV of 25%, and NPV of 96.3%. The positive likelihood ratio was 3.24, and the negative likelihood ratio was 0.373 (Table 4B).

**Table 3. Clinical data of pregnant women by gestational diabetes diagnosis (11-14 gestation weeks).**

| Variable | | Non GDM pregnancies 233 (90.7%) | GDM pregnancies 24 (9.3%) | p-value |
|---|---|---|---|---|
| Age (years), median (iqr) | | 30.0 (26.0; 34.0) | 32.0 (28.0; 36.0) | **0.03** |
| Pre-pregnancy previous smoke (%) | | | | |
| | No | 140 (88.6) | 18 (11.4) | |
| | yes | 93 (93.9) | 6 (6.1) | 0.15 |
| Pre-pregnancy alcohol intake (%) | | | | |
| | No | 232 (90.6) | 24 (9.4) | |
| | yes | 1 (100.0) | 0 (0.0) | 0.75 |
| Pre-pregnancy drugs use (%) | | | | |
| | No | 199 (90.9) | 20 (9.1) | |
| | yes | 34 (89.5) | 4 (10.5) | 0.79 |
| Weight (kgs.), median (iqr) | | 65.0 (58.6; 73.1) | 68.1 (61.1; 81.9) | 0.07 |
| Fasting glycemia (mg/dl), median (iqr) | | 82.0 (78.0; 85.0) | 88.0 (82.3; 92.0) | **<0.001** |
| BMI (Kg/m2), median (iqr) | | 25 (23; 28) | 28 (24; 32) | **0.02** |

| | | | | |
|---|---|---|---|---|
| *GDM, gestational diabetes mellitus; BMI, body mass index; ***(bold),** significant p-value (< 0.05, Mann-Whitney test). The results are expressed as percentages* (%), *median values, and interquartile ranges (IQR).* | | | | |

**Table 4. Association between the selected GCF-RNAs, BMI and fasting first trimester glycemia at 11-14 weeks of gestation, according to the diagnosis of gestational diabetes mellitus (GDM): multiple regression logistic model (A) and bootstrap estimation validation of the multivariate logistic regression model (B). OR: odds ratio; CI: confidence interval; BMI: body mass index.**

| A | | | B | | | | |
|---|---|---|---|---|---|---|---|
| Variable | or | p-value | Cl 95% | Variable | OR | p-value | IC 95% |
| BMI | 1.13 | 0.028 | 1.01 - 1.26 | BMI | 1.07 | 0.028 | 1.01 - 1.14 |
| Fasting | | | | Fasting | | | |
| Glycemia | 1.21 | <0.001 | 1.11- 1.33 | Glycemia | 1.12 | <0.001 | 1.06 -1.18 |
| TPA | 0.47 | 0.008 | 0.27 - 0.82 | TPA | 0.64 | 0.008 | 0.46 - 0.89 |
| PERP | 2.32 | 0.010 | 1.22 - 4.40 | PERP | 1.64 | 0.010 | 1.13 - 2.39 |
| NEAT-1 | 0.54 | 0.089 | 0.26 - 1.10 | NEAT-1 | 0.7 | 0.089 | 0.46 -1.06 |
| LINC-PINT | 1.86 | 0.048 | 1.01 - 3.43 | LINC-PINT | 1.44 | 0.048 | 1.01 - 2.06 |
| IL-1RN | 1.69 | 0.087 | 0.93 - 3.10 | IL-1RN | 1.36 | 0.087 | 0.96 -1.94 |

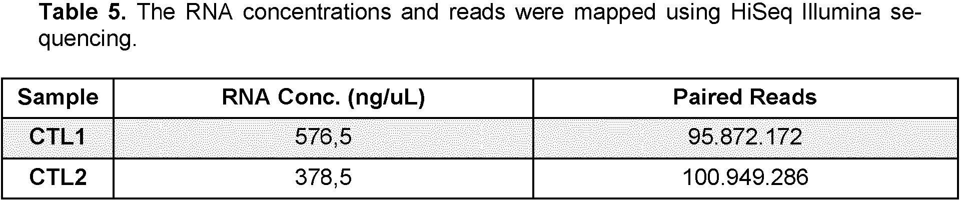

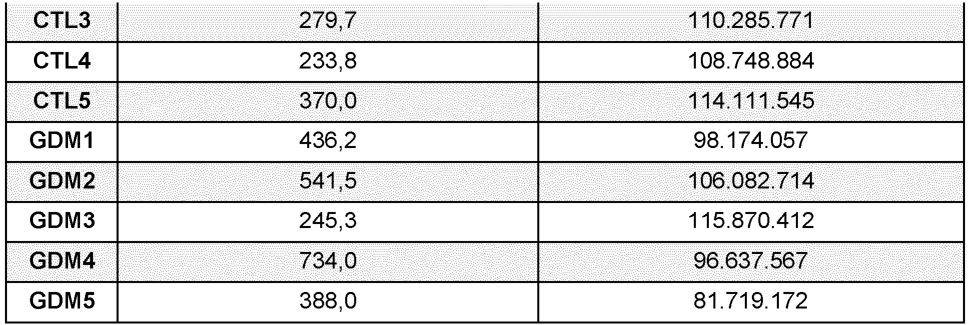

**Table 6.- IncRNAs candidates**

| **Up-Regulated** | **Down-Regulated** | |
|---|---|---|
| NEAT1 | LINC01166 | LOC285696 |
| LINC00673 | SUCLG2-AS1 | C15orf32 |
| MIR205HG | C9orf135-AS1 | LINC00960 |
| MALAT1 | FLJ33534 | LINC01604 |
| PP7080 | C2orf91 | FLJ16779 |
| RUNX1-IT1 | MORC2-AS1 | LINC01002 |
| LING-PINT | C7orf65 | CTBP1-AS |
| LINC00342 | TMEM105 | EPHA1-AS1 |
| LRRC75A-AS1 | SOCS2-AS1 | VPS9D1-AS1 |
| PSMA3-AS1 | C9orf139 | LOC729737 |
| RDH10-AS1 | EMX2OS | LINC01314 |
| PANDAR | RRN3P2 | LOC101928093 |
| | WDFY3-AS2 | LOC221946 |
| | LOC101929567 | LOC100506022 |
| | LOC100288152 | MAP3K14 |
| | LOC100129697 | LINC00324 |

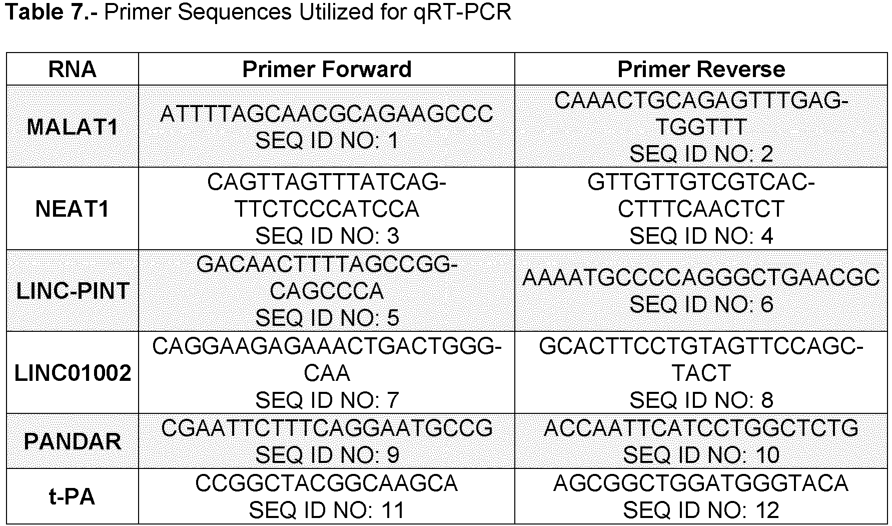

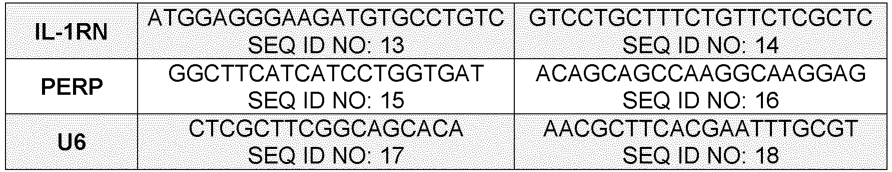

**Table 8: sequences of candidates RNA**

| | |
|---|---|
| cdna:lncRNA LINC-PINT | CGCCCACCGCGCGGAGGACAACTTTAGCCGGCAGCCCAGACCAGCGC GGCACCTGTCTCCGGAGTCTCCACCGCTCCTCCCGATTCATCCAGGGA AATTCTCAAGAATACGCTCTACAAATCTACGTGCGCATCATTTTCACCTC GCGTCGCGCCCGGGAGGAAGGAACGAGGCAAGGAGCTAAAGCAGCG TGCGTTCAGCCCTGGGGCATTTTATTAATGCTTTTACGAGTTAGAAGAG TTGGGATAATTTGCCATCTGGAGTTTCTCTGCCTTGCTGATCTGAGCTCA GACCTGCCAATTTACCAGAGATAATTGATAACACCCTGTAACAGCTGAG AGGAAAATGGAAGAAACGGAGATACTTTTAGTGAAGCAGAATAAACC ACTGAACAGGAAAAATGAGGAAGCTGTGAGTACCAGTGGAAGGAACG AGCCAGGAAGAGGGACTGGAACCATCTCAGAAGCCATGCCCCTCAGGC TGGAACTTGCCCTGTCTCCTCGCAGATGAGGTGCAGGTCTTCTGAACAA ATGCCTCCTGAGGATTTCTGTGCCTGGAAAATATCTGTTAACAGACTGC TTTTACACCTACCTACCTGTAAGAAGAATCAAGATAAAGAATTCATGAA TGATAAAAACACTTTGAAAAGACTGGTGGTTCCATAAAAGCTGACTTTG TGCCTGGAACATTGGAAGTGACCCGTCAGTGCTTACTGAATGAATAGG CAAATGAACAAAACCTCCTGCAATACAGCTTTCTTACCATGCCTCAAGA GACTCTTCAGTCCATCCTACTGTAGTTGCTTTTGATTGGGTTCTCCCTGA GTACTGACCCTAGGCCCAGATTCAGAGATTTCTCCAAAAAAAGAGCTTC TTTCAAAGACATCATTCCTATTGTCAACCCTGTTGGCATAGACACAAAAT GTAAACGTTGGACAAAGGCAATACTTGCTGTGACAAAGGCGATATTTG TTGTAACAAAACACAAATGTCTGTCCATCAGTGGGGAACGAGAATGCT GGGATTTGAAGAGGCCAAAAAAATAATACTGCTCTCTCTTTTGAGAAAG AGAGGGGATTTTTTTTTTTTTTTGAAAAGCCATAAGCTTAGGACAGAT GAAATTCAAAATCCACACTAAACTTCACTGGAGAATGTAAGCAAAATAT GAGGCCACAGGTGAGCTTTCTATTTCCTTCCTCCACTCTCCAAGATGGCC CATTGATATTTGGCTGTGTCCCCAAATCTCATCTTGAATTGTAGTTCCTG TAATCCCCATGTGTCATGGGAGGGATCCAGTGGGAGGTAATTGAATCA TGGGAGCAGTTACTCCCTTGCTGTTCTGTGATAGTGAATGAGATCTCAT GAGATCTGATGGTTTTATAAGGGCCTTTTTCCCCTTTGCTTGGCACTTCT CCGTCCTGCCATCATGTGAAGAGGGATGTGTTTGCTTCCCCTTCTGCCAT GATTGTAAGTTTCCTGAGGCCTCCCCAACCATGTGGAACTGTGAGTCAA TTAAACCTCTTTCTTTCATAAGTTA |
| SEQ ID NO: 19 | |
| cdna:lncRNA NEAT1 | GGAGTTAGCGACAGGGAGGGATGCGCGCCTGGGTGTAGTTGTGGGGG AGGAAGTGGCTAGCTCAGGGCTTCAGGGGACAGACAGGGAGAGATGA CTGAGTTAGATGAGACGAGGGGGCGGGCTGGGGGTGCGAGAAGGAA GCTTGGCAAGGAGACTAGGTCTAGGGGGACCACAGTGGGGCAGGCTG CATGGAAAATATCCGCAGGGTCCCCCAGGCAGAACAGCCACGCTCCAG GCCAGGCTGTCCCTACTGCCTGGTGGAGGGGGAACTTGACCTCTGGGA GGGCGCCGCTCTTGCATAGCTGAGCGAGCCCGGGTGCGCTGGTCTGTG TGGAAGGAGGAAGGCAGGGAGAGGTAGAAGGGGTGGAGGAGTCAG GAGGAATAGGCCGCAGCAGCCCTGGAAATGATCAGGAAGGCAGGCAG TGGGTGCAGGGCTGCAGGAGGGCCGGGAGGGCTAATCTTCAACTTGT CCATGCCAGCAGCCCCTTTTTTCCAGACCAAGGGCTGTGAACCCGCCT GGGGATGAGGCCTGGTCTTGTGGAACTGAACTTAGCTCGACGGGGCTG |
| SEQ ID NO: 20 | |
| | ACCGCTCTGGCCCAGGGTGGTATGTAATTTTCGCTCGGCCTGGACGG GGCCCAGGCCGGGCCCAGCCTGGTGGAGCGTCCAGGTCTGGGTGCGA AGCCAGGCCCCTGGGCGGAGGTGAGGGGTGGTCTGAGGAGTGATGTG GAGTTAAGGCGCCATCCTCACCGGTGACTGGTGCGGCACCTAGCATGT TTGACAGGCGGGGACTGCGAGGCACGCTGCTCGGGTGTTGGGGACAA CATTGACCAACGCTTTATTTCCAGGTGGCAGTGCTCCTTTTGGACTTTT CTCTAGGTTTGGCGCTAAACTCTTCTTGTGAGCTCACTCCACCCCTTCTT CCTCCCTTTAACTTATCCATTCACTTAAAACATTACCTGGTCATCTGGTAA GCCCGGGACAGTAAGCCGAGTGGCTGTTGGAGTCGGTATTGTTGGTAA TGGTGGAGGAAGAGAGGCCTTCCCGCTGAGGCTGGGGTGGGGCGGAT CGGTGTTGCTTGCCTGCAGAGAGGGTGGGGAGTGAATGTGCACCCTTG GGTGGGCCTGCAGCCATCCAGCTGAAAGTTACAAAATGCTTCATGGA CCGTGGTTTGTTACTATAGTGTTCCTCATGGCGAGCAGATGGAACCGG GAGACATGGAGTCCCTGGCCAGTGTGAGTCCTAGCATTGCAGGAGGG GAGACCCTGGAGGAGAGAGCCCGCCTCAATTGATGCCTGCAGATTGAA TTTCCAGAGGCTTAGGAGGAGGAAGTTCTCCAATGTTCTGTTTCCAGGC CTTGCTCAGGAAGCCCTGTATTCAGGAGGCTACCATTTAAAGTTTGCAG ATGAGCTTATGGGGGGCAATCTTAAAAAGTCCACAGCAGATGCATCCG GCTCGAGGGGCCATCAGCTTTGAATAAATGCTTGTTCCAGAGCCCATGA ATGCCAGCAGGCACCCCTCCTTTCCTGGGGTAAAGGTTTTCAGATGCTG CATCTTCTAAATTGAGCCTCCGGTCATACTAGTTTTGTGCTTGGAACCTT GCTTCAAGAAGATCCCTAAGCTGTAGAACATTTTAACGTTGATGCCACA ACGCAGATTGATGCCTTGTAGATGGAGCTTGCAGATGGAGCCCCGTGA CCTCTCACCTACCCACCTGTTTGCCTGCCTTCTTGTGCGTTTCTCGGAGA AGTTCTTAGCCTGATGAAATAACTTGGGGCGTTGAAGAGCTGTTTAATT TTAAATGCCTTAGACTGGGGATATATTAGAGGAAGCAGATTGTCAAATT AAGGGTGTCATTGTGTTGTGCTAAACGCTGGGAGGGTACAAGTTGGTC ATTCCTAAATCTGTGTGTGAGAAATGGCAGGTCTAGTTTGGGCATTGTG ATTGCATTGCAGATTACTAGGAGAAGGGAATGGTGGGTACACCGGTAG TGCTCTTTTGTTCTTGCTTCGTTTTTTAAACTTGAACTTTACTTCGTTAG ATTTCATAATACTTTCTTGGCATTCTAGTAAGAGGACCCTGAGGTGGGA GTTGTGGGGGACGGGGAGAAGGGGACAGCTTGGCACCGGTCCCGTGG GCGTTGCAGTGTGGGGGATGGGGGTATGCAGCTTGGCACTGGTACTG GGAGGGATGAGGGTGAAGAAGGGGAGAGGGTTGGTTAGAGATACAG TGTGGGTGGTGGGGGTGGTAGGAAATGCAGGTTGAAGGGAATTCTCT GGGGCTTTGGGGAATTTAGTGCGTGGGTGAGCCAAGAAAATACTAATT AATAATAGTAAGTTGTTAGTGTTGGTTAAGTTGTTGCTTGGAAGTGAGA AGTTGCTTAGAAACTTTCCAAAGTGCTTAGAACTTTAAGTGCAAACAGA CAAACTAACAAACAAAAATTGTTTTGCTTTGCTACAAGGTGGGGAAGAC TGAAGAAGTGTTAACTGAAAACAGGTGACACAGAGTCACCAGTTTTCC GAGAACCAAAGGGAGGGGTGTGTGATGCCATCTCACAGGCAGGGGAA ATGTCTTTACCAGCTTCCTCCTGGTGGCCAAGACAGCCTGTTTCAGAGG GTTGTTTTGTTTGGGGTGTGGGTGTTATCAAGTGAATTAGTCACTTGAA AGATGGGCGTCAGACTTGCATACGCAGCAGATCAGCATCCTTCGCTGC CCCTTAGCAACTTAGGTGGTTGATTTGAAACTGTGAAGGTGTGATTTTT TCAGGAGCTGGAAGTCTTAGAAAAGCCTTGTAAATGCCTATATTGTGG GCTTTTAACGTATTTAAGGGACCACTTAAGACGAGATTAGATGGGCTCT |
| | TCTGGATTTGTTCCTCATTTGTCACAGGTGTCTTGTGATTGAAAATCATG AGCGAAGTGAAATTGCATTGAATTTCAAGGGAATTTAGTATGTAAATCG TGCCTTAGAAACACATCTGTTGTCTTTTCTGTGTTTGGTCGATATTAATA ATGGCAAAATTTTTGCCTATCTAGTATCTTCAAATTGTAGTCTTTGTAAC AACCAAATAACCTTTTGTGGTCACTGTAAAATTAATATTTGGTAGACAG AATCCATGTACCTTTGCTAAGGTTAGAATGAATAATTTATTGTATTTTTA ATTTGAATGTTTGTGCTTTTTAAATGAGCCAAGACTAGAGGGGAAACTA TCACCTAAAATCAGTTTGGAAAACAAGACCTAAAAAGGGAAGGGGATG GGGATTGTGGGGAGAGAGTGGGCGAGGTGCCTTTACTACATGTGTGA TCTGAAAACCCTGCTTGGTTCTGAGCTGCGTCTATTGAATTGGTAAAGT AATACCAATGGCTTTTTATCATTTCCTTCTTCCCTTTAAGTTTCACTTGAA ATTTTAAAAATCATGGTTATTTTTATCGTTGGGATCTTTCTGTCTTCTGG GTTCCATTTTTTAAATGTTTAAAAATATGTTGACATGGTAGTTCAGTTCT TAACCAATGACTTGGGGATGATGCAAACAATTACTGTCGTTGGGATTTA GAGTGTATTAGTCACGCATGTATGGGGAAGTAGTCTCGGGTATGCTGT TGTGAAATTGAAACTGTAAAAGTAGATGGTTGAAAGTACTGGTATGTT GCTCTGTATGGTAAGAACTAATTCTGTTACGTCATGTACATAATTACTAA TCACTTTTCTTCCCCTTTACAGCACAAATAAAGTTTGAGTTCTAAACTCAT TAGAATTGTTGTATTGCTATGTTACATTTCTCGACCCCTATCACATTGCCT TCATAACGACTTTGGATGTATCTTCATATTGTAGATTTAGGTCTAGATTT GCTAGCTCCAAGTAATTAAGGCCATGTAGGAGAGCATGGTAACCACAG ATAGAACTGGTATTATCCCAAGTGGTCTGCAGACTGCTGAGTGGGGAT GGGATCTGCTCTCTGTTGAGAGTTGGTAATCATTGGTTTGAAATGTGAT GAAACCACTCAAGCCAATGAAGGTGGGTGTGTAGGTGGGGAGTACTTT GCCATAATATTTTAAAACATTACCTGGTTAGAGTTCTAAGTGGTACTTAT TTTTGTTTGGTTAGGGGAAAGCCTGAATAAAAACAGAAATGGACACAT AATATGCATATTCCATAGTCTTTGGGAGGCTGGAATGTGCCTGGGATTT GGGTCTAAGTGTATGCGTAATTCTTACCTCACTAAAGAATTTGCCTTGTT TTTTTCCTTTTGGTGAGTGACTAAAACGTCTGGGCTTCCCTGTGTGCGTG CTACAGTAAGCAAGCAGAGGCTGTGCAAAGGTGTGAGCAGGATCACG TGGAATCTGGAGGATACATCTTGGCTTGCAAACTGCCTCTGTCTCCTGG GTGGGACTGTTCTGTCCTTGCACTGCTGTTCTGTGTTACCTCTTGGGGT GTAAGGTTTTGCTTACAGGAGACAAACTTTGGGCGTAGAATGGAAGCC ACTGCCAGCCTCTGTGCTGAGAAGGAAGGTGCTTGTTTCAAAGGGAGC AGCAAGGGAGGCTTGTTCTACTCACCTGGGCCTGTTTGCCTGAGAAGG GGAGATAAGGGCTGAACTGGGACTAGCCAGGGGGACCAACACAAATG GTGGGGGATCATGACCTGAAGGATTCTTTCCTTCCCATGAGCTGCAGG GCTGGTTGCCGTCCTTGCAACTGTGTCTTATTTGCCTGTGCCGTTATATC TTGGTGACCCCTCCACGTGTACACTACTGACAAACGGGTGGAGTGCTG GGGAGAAGTCACTGTGCCGCCCACCTAGTAAACCTTCTGTCTGTGCTCA TGGCATCTCCAAGATGGGGCACTGCTGTGTGCAGAATCCAGGGTCCTC TTTCTGCTTGCAACTCCTTTCCCTGGATGCCCCAGAAACAATCCAGGCCT CCTTTCCTATCTTACCCCTTTGCTTTGCTTTTTACCCCAGCACCTCTATAAC CGCCTTCTCTTCTTTTCAGAACTCCTTGTTTCTCGTCCTGTTTTTTATGATT ACAAAACTCTTGCTTCCACCCTGGAAGATAACTGCTATAGATGCCTGTA TGTAAATGGTGCTGTCTCCAGCAACTGGCATGCTGAAGAAGAATTGATT CACGGGGTATAAATGTTGGGGATTGGAAGTGGGGATGAAATGGCACT |
| | TGTTGATACAGGAGCAGAGAGGTGAGGCCGACTGCTGAAGACAGCTC GCCACCCTCCTTGCCTCCACTCCAATCCAGGGGCTGGGGCCACATTCTTT GCCTTCATTTATCCTCAGATCAGGTGAGATCGACAGGAGGTGTTGATG GCAGTGCCAGCAATTATTGCTAATCCGTTTGCATCCTTATGCATAGATCT GAATTCAGACTTTGTGAATTTCCAGAGGTGTGGGTAATATAATAGAATT CAGTGAGTGGGCATGGCTGATCTTGTGCAAATTAAAAGTTATGGGGCA TAAGAATAGCAAAAGTTGAACTTCTTTAAAAAGGAAAGTACCCTGAG AGCCAGTATTGGTTGAGGCTCTTCAGTATGCCCAGGTTGGCAGCACTG AGAACCGCAGGAACGGCCTGTTGTTACAAAAAGGAGATTGACTCAGCT GCCCTTGGTGCATCTGACTGACTATGACTGCTGAGAGATTCCAAGGACC CTTAATGCCAGGGCTAACCTCTCCATGTGCAGTGAGACCTCTGGAGGA AGTGTCATCCTCTGGCTTTGTGTGGTACTCATTATGGTGCAGTGCGGGC ATGAAATGAAGACACCCAAATAGGCTTACAGATACGATATGTTTTAAAT GTTCGTATTTAACAAAAACATACTGACACTGTTTGGAAATGGCAACAGG AAGATAGCAAAATGAATACTAACATTACGAAAAGATGAACAGGTACAT GTTCCAAGGCAGGTGGCTGTGAACTTCCTCTGAGTGAAGGCATCCCCTC CAGCACCTTTCAGCCTGCTAGTTAGGACGACCCGCCGCCACCCTCCAGG ACCTCCAGCCCTGCACTGCCTTTCCTCTCTTTTAAATAATTCTTCATTGAG TTCTAATATGTAAAAAAAAAAAGTTTACTGTAAAGTTTGCAAATAAGGA AATTTTTTTTAAAAGTCCTCAGTAATCTTACCAGTAACAATTGTTATGGG CACATTTGCTTTTGGAAGATTTCTTTTGTATGCATGGGATAAGTACATTT TTAAACAAAAATGGGATTATGCCATAAATTCTATTTTGTGACTTTAATAT ATAGTGAACACCTTTTTTAATGATGACAGGATGTTCCCTTGCATGGCTG TATCAATTTAAACAATCTTGTTTCAATGGGCATACAGGGTATTTTCTAGT TTTTTTTTCCTCTTAGAAAATAATACTTGCGATGACTTTCCTTGTAGCTCA GACTTTTTCACGTCTGTTGTTATCTCTTTGGGAATGCTGAATACATACAT TTCGAGAAGGAAATGACTGTTAAACTCTTAAGACTTCAGGTTCATATTG CTAAACTGCCCAGCAGGGAGGGATTTTTTCAATTAGTGTTCTCACTGGT GAGGCAAACCTGATGCCTTCCCCTCTTCCTCAGAACCGGCTTTATCACAT TGAAAACCTTTGCTCCTCCGACGGATCGAGTCTGCTTTCCCTCTGGATGT GAGCATTGCTTTGTCTGCTGGTGACTGAACATCTCTACCTTGTGTCAATT GGCCATTTGTGGTGTGTGTGTGTGTGCGTGTGTGTGTGTGTGTGTGTGT ATGATTTTCTAATTCCTAGTCATTTTTCTATTGATTGTTTTGCAAAAGCCA TTTACATCTTAAGGATATTGATAATCTTTTGTTATATTTGATGCAAATATT TTTTTCCAGTTTATAGGTTGCCTTTTAATTTTGTGTTTCAGGTAGATAAA AGTTAAACGATTTTCTTAGGTTAGTTTATCACTGTGGTTTCTGAACTTGT TATGTGTAGATCTTTTCCACCCCAAGAGTACATAAATATTAATCCATACT TTCTTATGGAACTTGTATGGTTTCGTTTTTTACATTTAAACCTTCTTCCCC GTGGTGTGTGTTGTGGAATCTGTGTTTGTGTGAGGAGGGGCATGGTGC TCTCAGAACCCACCTCCTGTGGCCAGAGAGCCCTGTCCTGTGAGGGTG GTTGTCACAGTGGCAGGGTTCAATTCAGAAGACCTTGAGGGCAGGCTG ATGTTTCCTGAATGGGCCCCTGGTTGTTGCTTGTCCCTGACTCTCCATTT CCCCATCTGAGTGGATTTGGACCTAATAGGGCACTGGAGCTGGTTCGA ATCCTGACTGGACTACTTGGCAACTTTATGTCTGGGAGCAAGTTACTTA ACCTCCCCAAGCCTGTGTCTGTGAAATGCGGGTAAATGAATGTAGATGT TTGGCAGCAGCTACTCCTTGTTGAGCTCTCACAGTGAACTCTCCTGCCTC TGCCCTCCTTCCCCGCCTCCCCTGGTGCCTAGCGTCAGGTCTAGCCACTT |
| | CCTCCTGGGCCCCTCTCCCTTTTCTGTGGCTGGCTGCCTGCCCGCCTGGC GCTGGACCTTTCATGTAACGGAATCAGCATGTATATTCTGGTCTGGTC TGTTTCTACACTTAATTTTGTTTCCAGTAGTATTTCCCTGTACCGGCAGA GTTCACAAACACATTTGAAGAGGCTTTTCTCAGGATTCTTAACCTTCCC AAAGGAAGTCCCATGGATGGGTTTCTAGAAGTCTATAAATGCTCTGAA ATTGTATTTTTCTGTGGAAAGCATAACTTTCATCTGCTTGTTCGTGCTCA AAAAAGATCATGAATGAATGATTGCATGATTTATGCCATTGTGCTTAT ACTAAAGGATATGTAGCCCATCTCTTGAGCTGTTAAACTGTTTTGACTAC TTTAAATCGTGCAGCTGTGAGCATCTCTGTAAATTTAGTGTACACATGT ATCCCCTGGAGTGGCATTGCCTCGGCAGTGAGCACTTATGGTTTTATAA CTCTCTTCACAGACTCAAATGACTCCAGAAAGCTACACTTCCTGTTGTGA GTATATGATATCCATTTCCCTACATAGCCACTAACATCAGGTTTTACAA TTTTATTTATTTCTTGCTACTTTAAGAAATTTTTGTGGTGAAATACATATA ATAGAAGTTGACTATCTGAATCATTTTTAAGTATACATTCAGTAGTGTTA AGTATGTCGCCATTGTTGTACAACCAATCTCCAGAACTTTTTCATCTTGC AAAACAAACTCTGTACCCATTAAATAACATTAAACATTCCATTCCCTCCA GCCTCAGCAACCCCATTCTACTTTCTGTTTCTGTGAGTTTGACTATTCCA AGCACTTCATATCAGTTAAATCATGAAGTATTTGTCTGTCTGTGACTGGC TTATTTCTCTGAGCACAGTGTCCTCGAGATGCGTCTATGTTGTAGCATAT GTCAGAATTTCCTTCCTTTTTAAAAGATCCAAATAATATTCTTATTTTATA TCTTTTTTTTATCCATTCATCCATTAGTGGACACTTGGGTTGCTTTTGGCT ATTGTAAATAATGGTGCTATGTACAAATATCTATATTATTGTATTTACAA GTATAATGCTGTAATGTACACACATCTTTTTGAGATCCTACCTTCAGTTC TTTTGAGTATATAGCCAGAAGTGGTATTACTAAATCTTACGATATTTCTA TTTTTAATTTATTGAGGAACCACTGTAGTTTTTCATAGCAACTGCACCAT TTTACGTTCTCACCAAGAGTGCACAAGGGTTCCGAGGTTCCCACATCCT CCCCAACACTTGTTATTTTCTGCTTTTTTTAGATTGCAGCCATCATAGTG GGTGTGAGGTGACATTTCATTGTGGTTTTGATTTGCATTTCCCTAATGA GGAGTGATGCTGAGCATCTTTTCATATGCTTACTGGTCATTTGTATGTTG TCTTTGGAAAAATGTCTATTCAAGTCCTTTGACTATTTTAAAAATTGGGT TATTAGAGTTATCGTTGTTGTTGACTTGTAGGAGTTTCTTTCTATATTCT GGATATTAATCCCCTATCAGATATATGATTTGCAAATATCTTCTCTTATTC CATAAGGTTACTTTTTCACTTTGTTGATTGTGTTCTTTGATGTATAGAAG TTTTTAGTTTTGAAATAGTCTAATTTATCTGTTTTTACTTTTGTGGTCTGT GCTTTTGGTGTCATATCCAAGAAATCCTTGCCAAATCCAACGTTATAAG GTACTTTTAAGGTATTTTAGTTGTCTTAGTCTATATTTCTGTACTCACCTT TCTTTATCCACTCATCAGTTGATGGGCATGTAGGTTGGTTCCATATCTTT GCAATTCTGAATTGTGCTATGATCAGGTGTCTTTTTAGTATAATGATTTA CTCTCCTTTGGGTAGATACCCAGTAGTGGGATTGCTGGATCGAATGGTT TTTATAATTTTCTATTTTACCACAGTTTCTCTCTGCATTTTTCCTCTTTGAC CACTAACCATGTGAAATTCTCATATTGACCTTTATAATGATCATGAACTC TTAGTATCATTGGGAAGGCCACATTTGCCACTTATGATTGTAAACCTTAT CCTCCATTTTTCCTGTTATTGTTGGTGCAAAAAGCACCTATTATACCAGG ACTTTAAAAATCAGTCTGATAAGTCTTTGATAAGTCTAATAATAATAACT GATAAGTCCATTGAATTTGCTTCTGATTACTTTTTCTTTAGTAGCTAAAC ATGTATGTACTCCTATGATTACAATGAACACTCCTCTCCATTTAAATTAA TTATTTACATTGATGAAATAGCAAAATGTTAATGACTAAATACTGTCTTG |
| | GTTTTTTCGTTCCAGGTCAGTCAATATTAACTTCTTATAATTTCTTTTTTT TCTTTATGTGTGTGTGTGTGTGTATTTTTTTTTTTTAATTTCAATGGCTT TTGGGGTACAAATGGCTTTTGGTCATATAGATGAATTCTACAGTAGTGA AGTCTGAGATTTACTGCACCGGTCACCTGAGTAGTGTACATTGTACCC AATATGTGGTTTTTATACCTTGCCCCCTCTTACCCTCCCCACTTTGAGT CTCTAGTGTCCATTATGTCACTCTGTATACCTTTTGTACCCATAAGTTAG CTCTCACTTATAAGTGAGAACACACAGTATTTGGTTTTCCATTCCTGAGT TGCTTCACTTAGAATAATATCCTCCAGCTCCATCCAAAATTGCTGCAAAA AAAAAAAAAACCACAAACATTATTTGTTCTTTTTTATTGCTAAGTCATA TTCCATGGTGTAGAGATACCACATTTATTTATCCACTCACTGGTTGATG GGTTGGTTCCACATCTTTGCAATTGTGACTTGTACTGCCATCAAGTGTCT TTCTGGTATAATGACTTCTTTTCCTTTGGGTAGATACCCAGGAGTGGGA TTGCTAGATCAAATGGTTCTTAACATTTTCTCTCTGGATCTATTTCTGGA AATTTTAGGCTCCAGTTTTTGTTGTTGTTGTTAATAAAATGCAATGGAAT GTAATGATCATCACTTTTCATTATGCTTTAAAATCTGGTAAATGGAGGCT AGAACACTCCTGTAAGGCAAGAATATTCTCTCTGTTGGAACTCAAATAC ACAGAACTGGGTAAATCTCAATCTTAATCTTTGATTCAGGACACAACAT GGCTCTCTTTTACTTGCTTTCTTTAATTGTTTTTAATAATGTGGTAAGCA TTTCTGAATCTCCTATCCAATACAAAAACTAGGACAATACAGACAGTAA CTCCTATGGTTACAATGAACACTCCTCTCCACTTAAATTAATTATTTACAC TGATGAAATTGAAATAGCAAAATTTTAATGACTAAATACTGTCTTTGATT TTTTGTTCCAGGTCTGTCAATATTAACTTCTTATAATTTCTTTTTTTTTCT TTATGTGTGTGTGTGTGTGTATATATATATATTTAATTTCAATGGCTT TTGGGGTACAAATGGCTTTTGGTCATATATATGAGTTCTACAGTAGTGA AGTCTGAGATTTACTACACCTTCCACTTATGTGGTCCCACACCACCCGC CTCCCCTGCCGCCTCCTGCCACCCCCTAGGCCAAGGTAATAATCATCCTG AATCCTGGGTTTATCTCTCACTTGCTTTCTTTTCATATAATTTTGCAAAAG AATCTGATCTAAATGTGTTTTTCAGAGTATATATTTATATTTTAGCTGTTC TTAGAGAAAATTTATTATTTTGCATGTAATCTTATGGAACATTCTCATTT AATACCATGGTAAGATTCAGCCCTTGCCCAGGGGATAGTTCATTTAGTT TGTTTACTGGATAGAGCTCATCATGTGACTATACCTCAGTTAGTTTATCA GTTCTCCCATCCATGGTGACTAGGTTGCCTCTCAGCCTCTCAACAACACT GTTTCTCAGTGTCCTTGTAGAAGTGATATGTGGGTGTTTTCTCCTTACAC AGAGTTGAAAGGTGACGACAACAACGTTGGCACTACCAATCCCCCACC CTCCAGAGGGGTAACCAGTGTTACCAGTTTGCTGTGTTTCCTGCTACAC CTCGCCTTATTCACTTCCATTTGTATCTGAAAAACGTGTTGCATGGTTTC TTTTCTATAGAAGTGGTAAAATGCTATTGTGTCCTGTACATTATTGATTA CTTTTTTTCATTTAACAGTAGGGAGATGCCTGGGAGTACACAGAGAACT GCCCTCATTGTTTTCAACTTCTGCACTGTATGTCTGTGAGTTTAGCCATT CTGCTGTTAATGGAAATTTACAGTATTCTAATCTTTTGATATTACAAACA GTTCTGTGCGATCATCGTCATACACAACCCCTTGTGCACAATGCATGAG TGTTTCTCAGGGTAGGTACCAAGAAGTGAAATTCCTGGGTCATAGGGC GTGAGTCCGACATTTTTCTCCATTCTGCCCTGTTGCCCTCCAGAGTGGGT GTCCAGCTTTGCATACCTAAGTATGAGAGTATCTGTTGTTCATATCCTCT ACGACGCTCCATATATGAAACTTAAGTTTCTGCTAGTTGCCATCTTTGAT CTATCATGTATGCAGTGACCTACTAAGACTGTAATTGGTACAGTAGATT CTTGTCATCTGTGTGTGAATTTAGCATTCATGGGCTTAATGCTGACAAG |
| | GCCCCCAGGGTCCAAGACATATAATCATGTATAATTTTGTCAAGGTATA ATTTTTTAAATTGCTTTTGTCATGTGTCTGCTGGTGATGCCCAACCCAGT GCTCTGCACCCAGGTCACACTGTGGCTTTGTCCTCTGCTTATGCCTGCAT TGCAGCAACTGTCCTGAAGAGACCAAAATTATGCAGATTTAGGTAAGTC CATGGCTAATGTTATTATATTATGTGCTATTGTAATGGATGGGGCTGTG GAGTGTATGAATTTATAAATCACTGGTCTTGTAATTAAAATTCAAACACT ATAGAAAAAGGCCATGTAGAAGATAAAAGTTCCTCTATAATCCCGGAC CCCTAAGATAACTACTAATGACAACTTCATTTATATTCCTTCAGACATTTT CTGGCTGTGGATGTACTAAAATGTATCCTATTATTCTCTGCCCTAAAATG GAATCATACAAGGTGTACTGTTATTTTTATGGCTCTATAACATGTCATAT TGTACGTGTTGGTATGGTCATTTTAACCATTTTTCTAGTGATGGCTTTGA GGTTATTTGCAGTTTCCTAGCCATCTCAAAGTGTGCTGCGGGGATCTCT TTTGCATCCCTCTGGGTGCAGAGCTGAGGCACCCAGAGGCAGTGTCCA GAGGAGGCAGCATCTGTAGGTGTCTTCACCTGCTCTGGCTCTTGGCACA TCTGGTTGGTGACACTGTTTTGTGAGATGGGTTGAAAGCACGTGCTGCC AAAATAGAATAATGTTGGTCCTCTCCTCATGTGCCGTGGAACTGGGGTA AAACTGCGTAGTGGCTGCAGCTGCCTGTCCATACCGGAATCGAGTATA ACACGGTGCCTGGCTTAGCACAAAACAGTAGTGGGTCCTGCAGGCCCC AGAGTCTAATTCCTGGTATTCTTTCCCCTACACAGATTAAATAAACCAAA AACAAACTATTCTAGGAAAGCGTCTGTGACATTTGTAAAAAGTGGTATT TAATGATCTTTTATTCACTTGTCTGTTTAGTTTGTTGAAATCTTAAGTGGC ATCCTGGTCTGGGAAGGAGTGCTGTCTGCGCCTGCCCTCCGCTGGGCA CAGCGTGGCTGCTTCAGGGGCTAAGCACACACTTTCTGTCTTCTAAAGG GCCGCCACATGCCAGGAGCTCAGGTGTGAGCCCGGCTCTGGCTCTTAC CTCATAGGGTCACTCATAGGGGCACAGGGAGCAGAACATTGTACACAG CGAGGCACCACCCGGCTTGGCATCTGCCTCGGTGGACTTACTACCTCTA GAAGGAAATACCTGAGTTCCTCTGGCCTCAGCTCCTAGAGTGACTGGT GTGCTGTCCCTGTTACTCTTCTGTCAAGGTGACAACTGTGTGACCCATCA TCTGTGTGTCAAAGCAAGGCCCTGCCTGGGCCTCTGCTCCTGTGCTGAC CCCAAAGGCAAATGCTTTGCTAGTTTCCTTCCAGTTAATTTCACCTATGA ATAGATGTGTGAAAACTGTTCAAAGCCATACCTGCACATGTTTGAACTT CAAACCCTGTGGGTGATTCAGTGGCATCTTTCTCTAACCCCCAGCCTCCC TTCCCACAGAGGCCACCGTCATGGCCAGTTGCTGCAGTTTCTTTCCAGA GAACCTGTGTATGTGTAAAGCTGTACAGGCGTGGGTACACCACACAGC CTGTCTTGCACTGTGGACTGTTGAGTTACTAGTACATCTAGGTAAGCAC CGCATATCTGTATTCATGTCTGCCTTGGTCTTTTCAACATCTGTGTGGTA GCCGTGTTTGAATTACCCATTCCCTTTTTGGGGAACCATTAAGTTGTTTC AGCAATTTTTACTGTAGATAAGGCTATACCGCATATCTGTGTACATGGG TTTTTATGTACATGGGCAAGTATATCTGTGAGAGAAAAGTTTCCTCAGG AGGAATTCTGGGCACAGCATGTGTAAATTTCTAAATATGATGGACACCC CCAGCTTCCACCTCAAGGAGGTTGGTCCCATTGACATTTCCCCACACCTT CACCCAGGCTGTGCCCTTAAACTTGGTTATTTGTCAATGTGAGAAGTGG AAAATAGTATTTAATTGTAGTTTGGATTTGTATTTCTATTGGGTTGTATA CTTACTGATTAATAATAAGAGCTCTTTACATATTAAGGAAATTAACCCTT TTCAAATACATTCCTATTTCTCACTAATCTTTAAGTTTTATTGTAATATTTT GCTCTTTAGTTTATATATATATGTATATATATATATATGTATATATATATATA TATACATATATATATACATATATATATACTAATTTTCTTTTATGGTTCCTG |
| | GATTTTGTGAGTAGTTTGAAAAGGCTAATCCAGCTGAAGATTTTGTTGT TGTTGTTAAACCCCATGTTTCTCCTAACTCTTTTATTTTATTTGGAG GACTCTATCTAGACTTAATTTAGCATAACAAGTGACAGGGTTAGTTAG CCTGTTGTCCTTACACCATTTCTGGCTAATACAGCTATTAACTATTGATC TGTCTATTCACGTGCCAGTTCCTAATGGTTTACATAGTGTAATCTGCAC TTCAAAATAGCGAAGGGAAGCCCTACCTCATTATTCTACTTTCCAGAAT TCTCCTGGCTATTCCAGGCTGCATGTTTACCTTAACCTTCCCTGTGATGT CTTCATGCCGTTGTCTTCTTATGCAAGAATAAGGTACGTCTTTCCATCCA CTCACGTCTATTTAATTTGACTTTGCATTACACAGAAAGCTGGTCTTGGT CTGTCTACCTCGGCATCTAGTTGTCCTCACTGCCCCCTAGCCGACCCCAC CCCATCTGACTGACTACCCCATCACAGAGTACTTTTATTTACGTTTTGCT CTGCCTAATGGTTACTTGATACTGTCACGCCGACAGTGTCCAGTTCAGT GGTCTTTGCAGTTGAAATGCTCCCGTACACACTGTCTTGTTAAAAATGC CAGTAAGTTCATACAAACCCAGCTTGCACCCAAGGTCACATTCAGAGAG CGTAGGGCTGGGATGGGTTGTTTCCAAGCTTCTGCCACTGTGTGGCTA GCTCTTCCCACTGGGAAGTTCTGTGTACCCGGAATGTCGGAGTGGAGT CCTGTTCTAGTGTCCAGCACCTGACCCTGTGCCCAACCCCTCAACAGCCT ATTCCTGCTGTCCACAGCCTGCTGGAACTTTTTACAAAATATGTTGCCAT GCTGGACCCTGGGCACTGGACATAAGCCCCCTGGCAGCCTTTTTCATGT CACCCAAAGGGGTAATTGTCCTACTGGTGGTCTGTAAGATGAGTTAGG GTGACTTGCTAATAGACATTGTAAATCTTAATATTTATGTATGTATTTTA TTATTACCGGTTTTCCATTTATGATGGTAATATTGTTTCTTCTAAGAATAT TTATTTTTCCTTCTAAATATTGAGATAAAATTCATGCTTTTGAAATGTTCT ATTCAGTGGCTTTTAGTATATTTGCTATGTTGTGCAACCATCGACACTAT CCATTTCTAGAACTTTTTCGTCATCCCAAACAGACGCTCTGTATTCATAA AAAAATAACTTCCTACCTGTCTCTCCCCCTAGTCTTTGGTAACCTTTGTTA TACTGGTAAACTTTGTTGTGCTCTCTGTCTGTGTGAATTTGCCTATTCTA GGGGCCTCATATAAGTGTAATCATACAGTATTTGTCTTTTTGGGTCTGTC TGATTTCACTTAGCGGGTTTTCAGGGTTCATTCATGTTGCAGCATATAAC AGTACTGCGTTCCTTTTTCTGGCTGAATAATATTCCACTGTATGGATAGA CCCCATTTTGTTTATTCACACATCATTTGGACATTTGGATTATTTCTGGTT TTTGGCTATTATGAACAATGGTGCTATGAACAGTTGCGTACAAGTTTTT GTGTGAACATATGTTTTCAATTCTCTCATTATATACCTAGGAGTAGAATT ACTGGGTCATATGGTAACTGTATATTTTTGAGGAACTGCCAAACTATTTT CCCACGTCCATGCACCATTTCACATTCCCACCAGTAAGTAAGAGGGTTC CAATTTCTGCGCATTCTTGCCAACACTAGTTATTATCTGACTTTCTGGTTA TAATCATTCTAATGAGTGTGAAGTAGCCTCTGGTGTCATTTGGATTTGC ATTTCTCTGATGAGTGATGCTATCAAGCACCTTTGCTGGTGCTGTTGGC CATATGTGTATGTTCCCTGGAGAAGTGTCTGTGCTGAGCCTTGGCCCAC TTTTTAATTAGGCGTTTGTCTTTTTATTACTGAGTTGTAAGAGTTCTTTAT ATATTCTGGATTCTAGACCCTTATCAGATACATGGTTTGCAAATATTTTC TCCCATTCTGTGGGTTGTGTTTTCACTTTATCGATAATGTCCTTAGACAT ATAATAAATTTGTATTTTAAAAGTGACTTGATTTGGCTGTGCAAGGTGG CTCACGCTTGTAATCCCAGCACTTTGGGAGACTGAGGTGGGTGGATCA TATGAGGAGGCTAGGAGTTCGAGGTCAGCCTGGCCAGCATAGCGAAA ACTTGTCTCTACTAAAAATACAAAAATTAGTCAGGCATGGTGGTGCACG TCTGTAATACCAGCTTCTCAGGAGGCTGAGGCACGAGGATCACTTGAA |
| | CCCAGGAGGAGGAGGTTGCAGTGAGCTGAGATCATGCCAGGGCAACA GAATGAGACTTTGTTTAAAAAAAAAAAAAAGTGACTTGATTTAAGGGA AAAAATGACTGGCTATATTCAGTCAGATATGGCAAAAGTCTCAAGGT GTTAATGTGAATGATTAAGGTCTTGGGGGGGGTGTCCCCTATCAGACT ACAGGTGTTTAGAGGCACAGAAAAAGGTGCAGTTGGGTTCTTAATGTG AAATGATGAGAAGCACAACTCCAGTGTGTCTCTTTGTGTAGAATGTCAG CAGACACCCCCTGCTAGATGTGCTGGATCATGGGAAAGCATTTCCATTT GTTACTAGATTGTTCAGAAGTTTTAATTTATGATGGGTGTGGTGGCTCA TGCCTGTAGTCCCAGCACTGTGGGAGGCTGAGGCAGGAGGATCATCTG AGGCCAAGAGTTCAAGATCAGCCTGGGCAACATAGTGATACCCTATCTC TTAAAAAAGAAGAAGTTTTTAAATTTGAAATAATAATAGGTACTGGATT TATGCAAATGTCTTTTCTGCGTCTTTTGAGATGAGTATCAGGTTTTTTTT TTCCTTTTATCATCTGATGATGAACTTAATGTTTCCATTTGTATTAATGGA ATACTAAGTCCCTCTGTGATTTCTGAACCAAGCTATTCCTAGGCCTGAGT TTTATTTTGTTGACACAGAAATAAATTAGAAGGCCAAGCGTGGTGGCAT GTGCCTGTAGTCCTAGTTGCTGAGGTAAGAGGATTGCTTGAGCCCAGG AGTTCAAGGCTGCAGCAAGCTTTGATTGCGCCACTGCACTCCAGCCTTG GCGACAGACTAAGACGCTGTCTCAAAAAAAAACAAAAACGACAAAAAA AAAACAAAACAGAAAAAATAAACTAAGGCAATGACAGTCCCTGGCAAA TGCTGGGAGGGAGGCAGCAGTGGTCAGGGAAGGTAACCCTGAAGCAG GACTTGTAAAGCAAATAAGATTGGGAGGCCAAGGTGGGTGGATCACG AGGTCAGGAGTTCGAGACCAGCCTGGCCAACATAGTGAAACCCCGTCT TTACTAAAAATACAAAAAATTAGCCAGGTGTGGTGGGTGCCTGT AGTCCCAGCTACTTGGGAGGCTGAGGCAGGAGAATCTCGAACCCAGGA GGCGGAGGTTACAGTCAGCTGAGACCGCACCATTGCACTCCAGCCTGG GTGACAGAGCAAGATTCCGTCTCAAAAAAAAAAAAAAAAAAAAAAAACCA AGAAGAAAAGGAATGAATTAGAACTTCTTCTGCTTGGACTTAAGGGCA TCATCAGGCAGGTTTTGGGTAGGATAGCAGGGGAGGCAGAGACATAG TCGGGGTCAGTGGTCATGAGTGTGGCTTTGAGCCCAAAAACTTGGTTTC TGTTCCCTACTTTGCCACTCAGTAGTGCATGACTTTGGCCAAATTTCTTA AATTCATGAAGCAAGTTTCCGGGTGAATGAAATGGGGATAAAAATAGT GTTCAAACCTATCCGTTGGTTTGTGTGAAACTGAAATGAATAGTATCGT GCAGGTACTTGTGAGCAAGGGGAGCTGCTGTTTCCTGTCCCTTTATGAT GGGAAATATCTAGACAAGTTCCCAACCCTCTGCACTGCAGGCTGCATG GCACGGAGGGTCTTGTAACACCAGCTGGGGCTGGCCTTCTTTTAGGAG CTTCAGTGGTTCTGAAAACTTTTATTTGTTTGTTTGTTTTAGTAGATGTG GGGTCTTTCTGTGTTGCCCGGACTGGTCTCAAACTTCTGGACTCAAGTG ATCCTCCCCCGCTCAACCTCCCAAAGTGTTGGGATTACAGGTGTGAGCC ACTGTGCCCAGCCTTGAAAACTTTTTCAGGTTCTTCCAGGGTTACTGGG CTATTAAATATTTCTATTTCATTATAAGTCAGTTTTTCAAAGTTATATTAT CTTAATTACCTTTTTTATATGTATTAGTGTAGAGTAGCATTTTATATTTTG ATATCCTCCTTATGCATAGTTTTTCACTTTTTATTCCTAGTTTTTCGTTTTT AATAAGACTTTCAAGAAATTTATTTTATTGGCCTTTTGAAAAAAGCAGCT TTAGATAAAGTAAGCAGTTCTGCTTTCATTTTATAATTTATTTCTACTTTT GTTTCATTAATCTTTTCCTCCGGCATGCCTTGGATTTTGTTGTGTTACTCT TTTTCTAGAGGCTCGCATTGTGTGTCTGGTTCACTTATGATCACGCTTGC CTACTTTTAAGAATGGAAGAGGGGAGGTGGAGGGTGGCTGCACAGTC |
| | GAGGGTGTGAGGCAGTCTTGCTCTAGCCCCACCATGCCCTCAGCCCGCT GTGGCCACGCTGGTTCCTCAATTGCTGGGGCGTGCAGTGTCTGTAAGG GAGGCTACTGATGCCATCCGAGGAAGATGTAAGGTTTCGTGTGGGCAG CGAGAGCCTAGCAGGCATGTGGGGTGCCCAGCAAAGGGTAACAGTGG ACAGTTGTTGCCTCATTCCACAGAGTTTTGATTTTTTTTTTTTTTTAATG GTCACTCCATCAACATCCCCCATGGCCAGAGCCTGAGCTGGTCCCCAGA GACACAGGCATTCAGCTGACAGCCTCGCCTTCACGCTGCTGCTGTTCTC ATGGGGGACAGGCCTCAGGTGGCAATGCACAAATCATTAGTTAAGGGC AGTTGTGACAGTTACCAAGGAGTGTAGTCCCCCGCCCCCCGCCCAGTG AAAACAGCCCTAACCAGGGGTGGGGACCTTTGGGCTCTGACCCGAAGG GTAGGAGAAGCTGGAAGGACAGCATTCCTGTCTGCGAAGGCAGGAGC AAAGCTGCCAGGCTATGAAGGAAATGGCTGGAGCCTGAAGTCATGCAA GCTGGGGCTGGCAGGGACAGGGCCAACTTCCAGGCCTGGGGGCCACC ATGAGGATTCAGGACGTGACCCCCAGGGCACATGAAGGCCTTCCATCT GTATTTAAGAAAAGACTTTATCAGACGAGTATGGTGGCTCACGCCTGAA TCTTAGCACTTTGGGAGGCTGAGGCAGGTGGATCACGAGGTCAGGAGT TCAATACCAGCCTGGCCAATATGGTAAAACCCCATCTCTACTAAAACTA CAAAAATTAGCCAGGCATGGTGGCGCACGCCTGTAGTCCCAGCTACTC GGGAGGCTGAGGCAGAAGAATCACTTGAACCCGGGAGGTGGAGGTTA CAGTGAGCCAAGATCGCGCCACTACACTCCAGCCTGGGTGACAGAGTG AGACTCCGTCTCAAAAAAACCAAAAGACTTTATCTTATTTCCTATATGTT TGTGGTTTCAGTCCTGATGTATAATTTGACCCTAGTTAGAATGGTTATCT GAGGAAGTGGCCTGTACGATTTCTGCTTTTTAAATGTGTGGCTCCCTTT CTTCATTGATTAACGTATGATTATTTTTATAAATGTTCCATGGCAGTGGG AAGGGATTCTCTGTCACATTCCACATCTGGATCAGTTCCTCCCCATTTTG TTGGTCAAATCCGATCTGCCATATCCTGTGTAATGACAAGTGAGTTGCA TTCTCACCGTCACTCCTGGGGTCTCTCCGCTTCCCCTGAGCTGGCTCAGC AGTCTGCTCCATGTGTTTTGATGCAGGGTGACCCATTGGTATTCCCGAC ACTAACGCCCCCGTCTGTGGACTGCTTGCTGCTTGGGCTTCACTGTGTCT GGTGTTGACAGTGCAGACCTAAAGGTGTGCACACATGTGCACACACAC TCCGCTGTCTTCTTGTTTGCACTGGACTTAAATATCTATGAGGGTTATTT TCAACTGCTGAATTTGGAATGATTTTATATCTTTTCTGCTTTCTGCCCAT GTACATGTGTTTATTTTACACTGTTGTGATTGGTAGTTACTATGTGGGG ACACAATTACTTGGGCTGAAATAATCCACCTGTTGTGGTTGGGGTCCTC TGGGGCATTCCAGGGTGAGAGGTTGTCACTGCCACCTGGGCCATGTGG GCCGGCACCAGCATTTTGTGGTTACGAATTCTACAGTCACAAATATCTTT GGGCAAATCCCCTTCTATACCTCAAGGCAGCTTTTGGTTTGCAACCCCA CTGGCCAGAGGGAAGGGCCAGTCACTTGGCTCTCTCACTGCCCTGCGC CCCAGATGGTTCTAGGGCTGCTGTTTTCCCTTGGCCCTGCCAACACCACT GTTTTTACTTCTGCTCATTGGCTGAGTGCAGTGGTTCCTGGAAGCCAGT GGCACGTTTCCCCGCGTAGCTCGCTTATCCCACAGCACACACCCAAGGG TTCTGTTGCTAACACGCTGAATTAATTCTTTGCTCATCTTACAGAGTGTG TTTTGACTGCCCCCATTTCTGAGGCCTTGTAAGGCCAGAGCTTTGTTGCT TCATCGGCAGGTTGGGACTTAGATGGCCGTGAATGTTTCCTCTCTGCTG CTGCAGTAAGTAAGTGCCCGCACCATAGTGTGTTTGGAGGCTGAAGTT GAAGCGAGGCTGTGAGGGGAGATGGACGTGTGAGGAGGGATGATGG GGCTTGAGCAAAGTGGGGGAGGGGGCAAAGGCAGTTGGCCCAACACA |
| | TTCCCCACCCCTTTGAGAGGTCTGAGGCCTGCAGACCTGGCTCGGAGCC CACCTGGTAGTCCTCAGACTGTGTGTGTGTGTGTGTGTGTGTGTGTGTG TGTGTGTGTGTGTGTGTGTGTGTGTGTGTAAAAGAGAGAAGTTGTGGA GAAATGGGGGGCTGATTCTGCTCAGATTCATCAGGATGAGTAGAAGGC ACCCAGCTCTCACCCTGGCCTGACATGTGTGTCCCTGAGCAGGTTACAG TCCTCTCTGAGCCTCTGCTTCCCATCTGGACCCTGCTGGGCAGGGCTTCT GAGCTCCTTAGCACTAGCAGGAGGGGCTCCAGGGGCCCTCCCTCCATG GCAGCCAGGACAGGACTCTCAAATGAGGACAGCAGAGCTCGTGGGGG GCTCCCACGGACCCGCCGTGGGCCCAGGGGAGGCAGAGCCTGAGCCA ACAGCAGTGGTGCTGTGGACCGTGGATCCTGAGGGTGGCCTGGGGCA AGTACCGGCTGAGGGTCCAGGTGGGCTTTGTGTACCTTTGGGTCCTGG GGCCCTGGTGACTTGGACTCCAGGTTAGAGTCAAGTGACAGGAGAAA GGCTGGTGGGGCCCTGTGCTTCCGACTTCATTTCGAGTGATGGCAGTTC CCAGGAAGGAATCCACAGCTGACGGTGGCTGACAGATCAGAGAATGG AAGGCGAGGCAGGCGGGCGTCTGCGTGACCTCAGGTGCTTGGGGCCC AGCAGACCCAGAGAACCATTTCCACTAGGCCAGGGTGCCGGAAGTGTC CACAGGTCTTAGATTCCCTGTTCAGATGAAAAGATTTGTGCCTTTAATG ATAAAAGTGATCTGCATAGAGTCAAAAATTCAAGCCATGGGTATAAAA TGCAAGTAAAATCCCTGCCCTCACCTATCCCACCCTACTACACAGAGAT GTCCTCTCGAGTTTCCTAGACTCACTCTGGAAATTTCTGTATACACACAG AAGCTTGTGCCTCTGCTCGTGAAGGCAGAGGGAGGGAGAGCTGAAGG GCCAGCACCTTCTCACCTGTGGGCCCCCTCAGTGCTCGGTCCCAGAGCA TGCAGGACTGTGCCTCGTGTTCAGTTTGCTGGTCTGACTTCATGCTCCTT GGGCAGGATATGCATGTGCCATGCTAGGAGACATGTGGATGTGAAGCT GGGGGACAATGTCCCCTGGCTATGCCTTTACAAGGGAAGTAAGGAAG GTAGGAGGTGAGCCTGGGAGGGAGGGAGGGAGGCGCGGAGCCGCCG CAGGTGTTTCTTTTACTGAGTGCAGCCCATGGCCGCACTCAGGTTTTGC TTTTCACCTTCCCATCTGTGAAAGAGTGAGCAGGAAAAAGCAAAA |
| cdna:protein_c oding PERP | ACTCTCTGCTGTCGCCCGTCCCGCGCTCCTCCGACCCGCTCCGCTCCG CTCCGCTCGGCCCCGCGCCGCCCGTCAACATGATCCGCTGCGGCCTGGC CTGCGAGCGCTGCCGCTGGATCCTGCCCCTGCTCCTACTCAGCGCCATC GCCTTCGACATCATCGCGCTGGCCGGCCGCGGCTGGTTGCAGTCTAGC GACCACGGCCAGACGTCCTCGCTGTGGTGGAAATGCTCCCAAGAGGGC GGCGGCAGCGGGTCCTACGAGGAGGGCTGTCAGAGCCTCATGGAGTA CGCGTGGGGTAGAGCAGCGGCTGCCATGCTCTTCTGTGGCTTCATCATC CTGGTGATCTGTTTCATCCTCTCCTTCTTCGCCCTCTGTGGACCCCAGAT GCTTGTCTTCCTGAGAGTGATTGGAGGTCTCCTTGCCTTGGCTGCTGTG TTCCAGATCATCTCCCTGGTAATTTACCCCGTGAAGTACACCCAGACCTT CACCCTTCATGCCAACCCTGCTGTCACTTACATCTATAACTGGGCCTACG GCTTTGGGTGGGCAGCCACGATTATCCTGATTGGCTGTGCCTTCTTCTTC TGCTGCCTCCCCAACTACGAAGATGACCTTCTGGGCAATGCCAAGCCCA GGTACTTCTACACATCTGCCTAACTTGGGAATGAATGTGGGAGAAAATC GCTGCTGCTGAGATGGACTCCAGAAGAAGAAACTGTTTCTCCAGGCGA CTTTGAACCCATTTTTTGGCAGTGTTCATATTATTAAACTAGTCAAAAAT GCTAAAATAATTTGGGAGAAAATATTTTTTAAGTAGTGTTATAGTTTCAT GTTTATCTTTTATTATGTTTTGTGAAGTTGTGTCTTTTCACTAATTACCTA |
| SEQ ID NO: 21 | |
| | TACTATGCCAATATTTCCTTATATCTATCCATAACATTTATACTACATTTG TAAGAGAATATGCACGTGAAACTTAACACTTTATAAGGTAAAAATGAG GTTTCCAAGATTTAATAATCTGATCAAGTTCTTGTTATTTCCAAATAGAA TGGACTCGGTCTGTTAAGGGCTAAGGAGAAGAGGAAGATAAGGTTAA AAGTTGTTAATGACCAAACATTCTAAAAGAAATGCAAAAAAAAAGTTTA TTTTCAAGCCTTCGAACTATTTAAGGAAAGCAAAATCATTTCCTAAATGC ATATCATTTGTGAGAATTTCTCATTAATATCCTGAATCATTCATTTAGCT AAGGCTTCATGTTGACTCGATATGTCATCTAGGAAAGTACTATTTCATG GTCCAAACCTGTTGCCATAGTTGGTAAGGCTTTCCTTTAAGTGTGAAAT ATTTAGATGAAATTTTCTCTTTTAAAGTTCTTTATAGGGTTAGGGTGTGG GAAAATGCTATATTAATAAATCTGTAGTGTTTTGTGTTTATATGTTCAGA ACCAGAGTAGACTGGATTGAAAGATGGACTGGGTCTAATTTATCATGA CTGATAGATCTGGTTAAGTTGTGTAGTAAAGCATTAGGAGGGTCATTCT TGTCACAAAAGTGCCACTAAAACAGCCTCAGGAGAATAAATGACTTGCT TTTCTAAATCTCAGGTTTATCTGGGCTCTATCATATAGACAGGCTTCTGA TAGTTTGCAACTGTAAGCAGAAACCTACATATAGTTAAAATCCTGGTCT TTCTTGGTAAACAGATTTTAAATGTCTGATATAAAACATGCCACAGGAG AATTCGGGGATTTGAGTTTCTCTGAATAGCATATATATGATGCATCGGA TAGGTCATTATGATTTTTTACCATTTCGACTTACATAATGAAAACCAATT CATTTTAAATATCAGATTATTATTTTGTAAGTTGTGGAAAAAGCTAATTG TAGTTTTCATTATGAAGTTTTCCCAATAAACCAGGTATTCTAAACTTGTT TCCAGTTTGTAGTTTTTCCATTTTTCAAATCTGGGGAAAGGAATTAAAAA AAAAATGGGTAATAAGAACATGGGATATAATGAAAAGTGGTTTTTGTT TGTTTTTTTGTTTGAAGTTTTAAGGGCCTTGCTCATTTTAGGTGTCCAAA ACCAATTTTTGAGTGGAGATTAATGAATTCTAATAGTCTATTCCCTGAAC TTTTCCTCAATGAACAATACCCTAGACACACATTAAACAATTTCTCTGCA GTGCTATCAACCAGAGGAAAATGGACTAAGAGATTTCTGGCAGGTTCA GACACCCGGGGGACATGTGTGCAGTGTAGCTGAAGCCTCCTCCTTGTG CTGGGGTCCCCTTCCATTCAGGTGGTGGGGTAGCAGTCTCTCTATTTTC CCCTTGCCCTCCTTCCCATTTTATCATTTGTTATTTTTTTTCCCACCATAAG TCATATGTTACTTCCACTATGGTGTATGTCATTGTGAGGATGGGTGCAG AGAGGCTGGGTGGGAGAACGGAAATATATCTCCCTAGGGCTACTGTTG GCCAGCTAGTCCTTGGCAGTGAATTTTTCTATGCTTTTCAAAATGCGAG GTGAATGTTTCTCATAGAGAAATGTAATCTGGGTGATTATACCAAAATT GAAAAGAAAAACCCACACAACTATGCCGTGGCTGGTGGAGAATTTGAA GTGGTCATTAAAAATGTTAAAAATCCCATCTTTTAAAGTGATACCACAG CTCATTCAAGAAGATACTGGATATCTAGAGATTAAGAAACGTGGTCTCC TGTTAAACATGAAAATGACTCCGTTTATAAGCTTCTCTACCACATGCACT TGTCTTTGCATGATTTCCCATCCAGCCTTCTTCCCCTCCTCAATCACACAA TACCTTAACGGCGCACATTTAGGAAAAATGCAACCTCCTGGGACCAACG AGCCTGATATAATAGAACCATGTCAACCTAAAGTATTTATGACAAAGAT AAACTCTTATTTTGCAGAAATGGTCTGCTTCCTTCAGCCTTGTTCTAGTA TAGAGATCTGCCATTCCTTGTTGATCCAGATTCACCAAGACAGATACCTT TATGTCATAACAGAAGGGAAGTTCCAGAGGATTCTGGAGAGTAATGAA GAATTGGGCTGAGAAACCACCTGAAGGCTAACAGTGCATTGCATGAGA TTTCCCACAGTAAAGCTGAGGTGCTTTTTGGTTCAGTAATTAAATATTGA GTTCCCACCCTTTAAATAAGCAGTTCTAGGTTCCTAAGCAATTATTTCAC |
| | TCTGTAAGTAGCCAGACATGCTAAGTGGCACTTACTGCTGATTGTAACA AAGAAGTAATATATCAAGGTCTTTCCATGTTCACACAAGGTAGCTTGTG TGTAATAACTTAGCTTCAAAACCATAGACTGCAGAACTCACAAGTTCAA CAGCCTTTCCTTTTTTAAGGAAATGAAAACAATGGAAAATATAGTCATC ATAACTTAATTCGGTTTATTTTTTTTTCTGTAAACTCCCCCTGAAAGACA TTCCTATTAATACAGTAAATGTGAACACTGACTTGTTTTATAAGCACAT CTGAAAGGGCATATTTGAGTCTCATCCCAACTTTGGTCCTTGCTATCTGT GCAGGCTTGGGCAGGTCATCTCCCTGCTGGTCTCAATATCCTCACCTGT AAAATGATTGTAAATGATCCCCCTACCTTCAAGATTCTCTGATTGATAGA ATTTTTTCTTTAATTAAAAAATTTTAAATATTCCTTGAGTTGGAAGCACT GATCAATAAGTGGATTGCTTAGGGAGGTTGGAACGAATAGATTCAGTC CCAACTTCCTCTTTTAAATTCCCTCTTCCTCACTCTTCCTGCAACACTTATT TTTACAGTTGAGTTTTAAAAATAAGTAATATATAAAATAATTTCTGTAGT GTGGTTTCAGATTTAAAAATTCCTGCAGACAGGCTGGGCTTGCAACCCC ATCAGTCGATGGTCAGAGCCCTTTGCTTTTTGAGACCATTTTTAGGTGA GCTTGGCTTGCCTGGATACAGTGTGCAGTGCATTCTTCCTGAATTTTGC AATTCTGGTATCTGGGTGTATTTTCTAGGTGTGTCAGGGTGAGTGTAAT CCACCTAGGGTGTGGAAAAAGCCAAGAAAGGGAAATTAAAAGAGGTT CCTATCCAGTCATGTTAATGATCTTCCACTTGTACTATCCTGTGCTTCGTT GTTAACCTCGAAAACATACTTTGTTGGCTGCAAAAATAAACAAAGGGA AACTCA |
| cdna:protein_c oding tPA | AGAGCTGAGATCCTACAGGAGTCCAGGGCTGGAGAGAAAACCTCTGC GAGGAAAGGGAAGGAGCAAGCCGTGAATTTAAGGGACGCTGTGAAGC AATCATGGATGCAATGAAGAGAGGGCTCTGCTGTGTGCTGCTGCTGTG TGGAGCAGTCTTCGTTTCGCCCAGCCAGGAAATCCATGCCCGATTCAGA AGAGGAGCCAGATCTTACCAAGTGATCTGCAGAGATGAAAAAACGCAG ATGATATACCAGCAACATCAGTCATGGCTGCGCCCTGTGCTCAGAAGCA ACCGGGTGGAATATTGCTGGTGCAACAGTGGCAGGGCACAGTGCCACT CAGTGCCTGTCAAAAGTTGCAGCGAGCCAAGGTGTTTCAACGGGGGCA CCTGCCAGCAGGCCCTGTACTTCTCAGATTTCGTGTGCCAGTGCCCCGA AGGATTTGCTGGGAAGTGCTGTGAAATAGATACCAGGGCCACGTGCTA CGAGGACCAGGGCATCAGCTACAGGGGCACGTGGAGCACAGCGGAGA GTGGCGCCGAGTGCACCAACTGGAACAGCAGCGCGTTGGCCCAGAAG CCCTACAGCGGGCGGAGGCCAGACGCCATCAGGCTGGGCCTGGGGAA CCACAACTACTGCAGAAACCCAGATCGAGACTCAAAGCCCTGGTGCTAC GTCTTTAAGGCGGGGAAGTACAGCTCAGAGTTCTGCAGCACCCCTGCC TGCTCTGAGGGAAACAGTGACTGCTACTTTGGGAATGGGTCAGCCTAC CGTGGCACGCACAGCCTCACCGAGTCGGGTGCCTCCTGCCTCCCGTGG AATTCCATGATCCTGATAGGCAAGGTTTACACAGCACAGAACCCCAGTG CCCAGGCACTGGGCCTGGGCAAACATAATTACTGCCGGAATCCTGATG GGGATGCCAAGCCCTGGTGCCACGTGCTGAAGAACCGCAGGCTGACGT GGGAGTACTGTGATGTGCCCTCCTGCTCCACCTGCGGCCTGAGACAGT ACAGCCAGCCTCAGTTTCGCATCAAAGGAGGGCTCTTCGCCGACATCGC CTCCCACCCCTGGCAGGCTGCCATCTTTGCCAAGCACAGGAGGTCGCCC GGAGAGCGGTTCCTGTGCGGGGGCATACTCATCAGCTCCTGCTGGATT CTCTCTGCCGCCCACTGCTTCCAGGAGAGGTTTCCGCCCCACCACCTGA |
| SEQ ID NO: 22 | |
| | CGGTGATCTTGGGCAGAACATACCGGGTGGTCCCTGGCGAGGAGGAG CAGAAATTTGAAGTCGAAAAATACATTGTCCATAAGGAATTCGATGATG ACACTTACGACAATGACATTGCGCTGCTGCAGCTGAAATCGGATTCGTC CCGCTGTGCCCAGGAGAGCAGCGTGGTCCGCACTGTGTGCCTTCCCCC GGCGGACCTGCAGCTGCCGGACTGGACGGAGTGTGAGCTCTCCGGCTA CGGCAAGCATGAGGCCTTGTCTCCTTTCTATTCGGAGCGGCTGAAGGA GGCTCATGTCAGACTGTACCCATCCAGCCGCTGCACATCACAACATTTA CTTAACAGAACAGTCACCGACAACATGCTGTGTGCTGGAGACACTCGG AGCGGCGGGCCCCAGGCAAACTTGCACGACGCCTGCCAGGGCGATTC GGGAGGCCCCCTGGTGTGTCTGAACGATGGCCGCATGACTTTGGTGGG CATCATCAGCTGGGGCCTGGGCTGTGGACAGAAGGATGTCCCGGGTGT GTACACCAAGGTTACCAACTACCTAGACTGGATTCGTGACAACATGCGA CCGTGACCAGGAACACCCGACTCCTCAAAAGCAAATGAGATCCCGCCTC TTCTTCTTCAGAAGACACTGCAAAGGCGCAGTGCTTCTCTACAGACTTCT CCAGACCCACCACACCGCAGAAGCGGGACGAGACCCTACAGGAGAGG GAAGAGTGCATTTTCCCAGATACTTCCCATTTTGGAAGTTTTCAGGACTT GGTCTGATTTCAGGATACTCTGTCAGATGGGAAGACATGAATGCACAC TAGCCTCTCCAGGAATGCCTCCTCCCTGGGCAGAAAGTGGCCATGCCAC CCTGTTTTCAGCTAAAGCCCAACCTCCTGACCTGTCACCGTGAGCAGCTT TGGAAACAGGACCACAAAAATGAAAGCATGTCTCAATAGTAAAAGATA ACAAGATCTTTCAGGAAAGACGGATTGCATTAGAAATAGACAGTATATT TATAGTCACAAGAGCCCAGCAGGGCCTCAAAGTTGGGGCAGGCTGGCT GGCCCGTCATGTTCCTCAAAAGCACCCTTGACGTCAAGTCTCCTTCCCCT TTCCCCACTCCCTGGCTCTCAGAAGGTATTCCTTTTGTGTACAGTGTGTA AAGTGTAAATCCTTTTTCTTTATAAACTTTAGAGTAGCATGAGAGAATT GTATCATTTGAACAACTAGGCTTCAGCATATTTATAGCAATCCATGTTAG TTTTTACTTTCTGTTGCCACAACCCTGTTTTATACTGTACTTAATAAATTC AGATATATTTTTCACAGTTTTTCCAAAATCAGAGTGGAATGGTTTTGTTA TAGATGCTGTATCCCACTCTTTATTCATGTTCACATTTTAAAATCATTTGG AATTCTGCTTCACTCGCTTAACATATACACAACACCTGTAACATACAAGG CAATGGGCTAGGTGCTCCAGACCGGGAAAAGGAGGGACAGGAATGCT TGGTCTGATGGGCTAATATGGCATTTAGAGAAGTACCAAGGTACAGTG GAGCCGGTCACAAAAGGGCAGACTTGTAGTAGAATTCAGTTGCAAGAG GGATTGGGGAATCTTAAGGAAAAAATAGAATCTTAAGGAAAAAATAAC TGGGTGAGACGTGGACTGTGGACAGGTGTGGAAAAGGCACTCTCCAT GGAGGTATGAATATGTAGAGGGCCAAGAGAGGGGAGTACAGGGAGA AATGAGTTGAGCTTGTCTGAAGTGAACTTCAGGAAGAGGAACATAGGC TGGAATTTAGATTATGGGGGCTCTGAACACCAAACTGAGTTTGGACTTA ATTGACTTCTG |
| cdna:protein_c oding IL-1RN | AGTCCACTGCCTTGCTGCAGTCACAGAATGGAAATCTGCAGAGGCCTCC GCAGTCACCTAATCACTCTCCTCCTCTTCCTGTTCCATTCAGAGACGATC TGCCGACCCTCTGGGAGAAAATCCAGCAAGATGCAAGCCTTCAGAATC TGGGATGTTAACCAGAAGACCTTCTATCTGAGGAACAACCAACTAGTTG CTGGATACTTGCAAGGACCAAATGTCAATTTAGAAGAAAAGATAGATG TGGTACCCATTGAGCCTCATGCTCTGTTCTTGGGAATCCATGGAGGGAA |
| SEQ ID NO: 23 | |
| | GATGTGCCTGTCCTGTGTCAAGTCTGGTGATGAGACCAGACTCCAGCTG GAGGCAGTTAACATCACTGACCTGAGCGAGAACAGAAAGCAGGACAA GCGCTTCGCCTTCATCCGCTCAGACAGTGGCCCCACCACCAGTTTTGAG TCTGCCGCCTGCCCCGGTTGGTTCCTCTGCACAGCGATGGAAGCTGACC AGCCCGTCAGCCTCACCAATATGCCTGACGAAGGCGTCATGGTCACCAA ATTCTACTTCCAGGAGGACGAGTAGTACTGCCCAGGCCTGCCTGTTCCC ATTCTTGCATGGCAAGGACTGCAGGGACTGCCAGTCCCCCTGCCCCAG GGCTCCCGGCTATGGGGGCACTGAGGACCAGCCATTGAGGGGTGGAC CCTCAGAAGGCGTCACAACAACCTGGTCACAGGACTCTGCCTCCTCTTC AACTGACCAGCCTCCATGCTGCCTCCAGAATGGTCTTTCTAATGTGTGA ATCAGAGCACAGCAGCCCCTGCACAAAGCCCTTCCATGTCGCCTCTGCA TTCAGGATCAAACCCCGACCACCTGCCCAACCTGCTCTCCTCTTGCCACT GCCTCTTCCTCCCTCATTCCACCTTCCCATGCCCTGGATCCATCAGGCCA CTTGATGACCCCCAACCAAGTGGCTCCCACACCCTGTTTACAAAAAAG AAAAGACCAGTCCATGAGGGAGGTTTTTAAGGGTTTGTGGAAAATGAA AATTAGGATTTCATGATTTTTTTTTTCAGTCCCCGTGAAGGAGAGCCCT TCATTTGGAGATTATGTTCTTTCGGGGAGAGGCTGAGGACTTAAAATAT TCCTGCATTTGTGAAATGATGGTGAAAGTAAGTGGTAGCTTTTCCCTTC TTTTTCTTCTTTTTTTGTGATGTCCCAACTTGTAAAAATTAAAAGTTATGG TACTATGTTAGCCCCATAATTTTTTTTTTCCTTTTAAAACACTTCCATAAT CTGGACTCCTCTGTCCAGGCACTGCTGCCCAGCCTCCAAGCTCCATCTCC ACTCCAGATTTTTTACAGCTGCCTGCAGTACTTTACCTCCTATCAGAAGT TTCTCAGCTCCCAAGGCTCTGAGCAAATGTGGCTCCTGGGGGTTCTTTC TTCCTCTGCTGAAGGAATAAATTGCTCCTTGACATTGTAGAGCTTCTGG CACTTGGAGACTTGTATGAAAGATGGCTGTGCCTCTGCCTGTCTCCCCC ACCGGGCTGGGAGCTCTGCAGAGCAGGAAACATGACTCGTATATGTCT CAGGTCCCTGCAGGGCCAAGCACCTAGCCTCGCTCTTGGCAGGTACTCA GCGAATGAATGCTGTATATGTTGGGTGCAAAGTTCCCTACTTCCTGTGA CTTCAGCTCTGTTTTACAATAAAATCTTGAAAATGCC |

## Claims

1. *In vitro* method for screening or identifying pregnant females at risk of developing gestational diabetes mellitus (GDM), the method comprising the steps of:
(a) measuring the nucleic acid expression levels of one or more RNAs in a gingival crevicular fluid (GCF) sample obtained from a pregnant female to be screened, and
(b) comparing the RNA expression levels obtained in (a) with the expression levels of the same one or more RNA measured in a control GCF sample and determining that the pregnant female screened in (a) is at risk of having gestational diabetes mellitus if the RNA expression levels measured in (a) are deviated or different compared to the RNA expression levels measured in the control GCF sample.

2. The *in vitro* method of claim 1, wherein the one or more RNAs is selected from the list of Tables 1 or 6 or 8.

3. The *in vitro* method of claim 1 or 2 wherein the one or more RNAs is selected from the list consisting of LINC-PINT, NEAT1, TPA, PERP, or IL-1RN, and wherein the pregnant female to be screened is identified as being at risk of having gestational diabetes mellitus if the RNA expression levels measured in (a) are increased in comparison to the expression levels of said RNA measured in a control GCF sample.

4. The *in vitro* method of claim 3, wherein the one or more RNAs LINC-PINT, NEAT1, TPA, PERP, and IL-1RN comprise a nucleotide sequence as set forth in SEQ ID NO: 19, 20, 22, 21, 23, respectively.

5. The *in vitro* method of any one of claims 1 to 4, wherein the one or more RNAs are the long noncoding RNAs (ncRNAs) NEAT1 and/or LINC-PINT.

6. The *in vitro* method of any one of claims 1 to 5, wherein the pregnant female to be screened is the first trimester of pregnancy, preferably in 11-14 weeks of pregnancy.

7. The in vitro method of any one of claims 1 to 6, wherein the control GCF sample is a GCF sample obtained from a female or from a population of females that did not develop gestational diabetes mellitus during and/or after pregnancy.

8. The *in vitro* method of any one of claims 1 to 7, wherein the risk of having gestational diabetes mellitus is determined in step (b) when the one or more RNA expression levels measured in (a) are:
- at least a base 2 log fold change of ≥1 or ≤-1 than the same one or more RNA expression levels measured in a control GCF sample, and/or
- statistically significant higher or lower than the same one or more RNA expression levels measured in a control GCF sample, wherein the p value is < 0.05.

9. The *in vitro* method of any one of claims 1 to 8, comprising a further step (c) of treating the pregnant female screened if step (b) determined that she is at risk of developing gestational diabetes mellitus.

10. Method for obtaining useful data for the *in vitro* diagnosis of gestational diabetes mellitus comprising the steps defined in any of claims 1 to 9.

11. A kit comprising one or more biomarker detecting reagents for measuring in a gingival crevicular fluid (GCF) sample obtained from a pregnant female the ncRNA expression levels of one or more RNAs selected from Table 1 or 6 or 8, preferably selected from the list consisting of LINC-PINT, NEAT1, TPA, PERP, and IL-1RN.

12. Use of a kit for screening for pregnant females at risk of developing gestational diabetes mellitus, the kit comprising one or more biomarker detecting reagents for determining a differential expression level of one or more RNAs selected from Table 1 or 6, preferably selected from the list consisting of LINC-PINT, NEAT1, TPA, PERP, or IL-1RN, and wherein a deviation in the expression levels of at least one of said ncRNAs in comparison to a control GCF sample is indicative that the pregnant female is at risk of having gestational diabetes mellitus.
